# EUROPEAN PATENT APPLICATION

(11) **EP 2 372 591 A2**
(43) Date of publication of application: **05.10.2011**
(21) Application number: 11003233.1
(22) Date of filing: 15.06.2007
(51) Int. Cl.: G06F 19/00

(54) **System and method for collecting patient information from wich diabetes therapy may be determined**

(30) Priority: 16.06.2006 US 424757
(62) Divisional of application: 07796156.3
(71) Applicant: Roche Diagnostics GmbH, 68305 Mannheim (DE); F.Hoffmann-La Roche AG, 4070 Basel (CH)
(72) Inventor: Essenpreis, Matthias, Dr., 69469 Weinheim (DE); Jeckelmann, Joel, 1752 Villars-sur-Glâne (CH); Bernini, Nicole, 3400 Burgdorf (CH); Meyer-Olden, Gunnar, 26180 Rastede (DE); Wagner, Robin, Indianapolis IN 46220 (US); LaBastide, Sebastian, San Mateo CA 94402 (US); Schoemaker, Michael, Dr., 69163 Mannheim (DE); Weinert, Stefan, Pendleton IN 46064 (US); Heaton, Kelly, Millwood VA 22646 (US); Brandt, Derek, 4436 Oberwil (CH)
(74) Representative: Silber, Anton

(57) **Abstract**

A system is provided for collecting patient information from which diabetes therapy may be determined. The system may comprise a patient interface device, a patient notification device, an input device for entering patient information, and an information collecting unit. The information collecting unit may include a processor electrically coupled to a memory having stored therein at least one algorithm executable by the processor to activate the patient notification device followed by presenting instructions to the patient via the patient interface device for collecting specified information from the patient via the input device.

## Description

### CROSS REFERENCE TO RELATED APPLICATIONS

This application claims the benefit, under 35 U.S.C.119(e), of U.S. Patent Application No. 11/424,757 which was filed March 16, 2006 and which is hereby incorporated by reference herein.

### FIELD OF THE INVENTION:

The present invention relates generally to diabetes care, and more specifically to techniques for collecting patient information from which diabetes therapy may be determined.

### BACKGROUND

The type and intensity of therapy needed by a patient with diabetes typically varies according to the patient's lifestyle including, for example, the patient's weight, age, eating habits, physical activity, overall health, stress level, and the like. It is desirable to collect histories of such lifestyle information from patients so that this information may be used to determine and prescribe appropriate diabetes therapies. The content and detail of such lifestyle information that is needed typically varies from patient to patient, and it may therefore be further desirable to design the collection of such lifestyle information on a patient-by-patient basis.

### SUMMARY

The present invention may comprise one or more of the features recited in the attached claims, and/or one or more of the following features and combinations thereof. A system for collecting patient information from which diabetes therapy may be determined may comprise a patient interface device, an input device for entering patient information, and an information collecting unit. The information collecting unit may include a processor electrically coupled to a memory having stored therein at least one algorithm executable by the processor to present instructions to the patient via the patient interface device for collecting the patient information from the patient via the input device. The patient information may include information relating to timing and carbohydrate amount of food consumed by the patient, the composition of food consumed by the patient, insulin received by the patient, therapy related medication being taken by the patient and/or the physical state of the patient.

The system may further include a glucose measuring device configured to measure glucose concentration of a body fluid of the patient. In this case, the patient information may further include measurements of the patient's glucose concentration via the glucose measuring device. Alternatively, the system may further include a glucose measuring device configured to measure glucose concentration of a body fluid of the patient and produce a corresponding glucose concentration value, and means for automatically transferring the glucose concentration value to the information collecting unit. In either case, the system may further include an insulin infusion device configured to be responsive to an infusion command to infuse a corresponding quantity of insulin into the body of the patient, and means for automatically transferring the insulin quantity to the information collecting unit.

The patient interface device and the input device may illustratively each be carried by the information collecting unit and may each be electrically connected to the processor. In this embodiment, the information collecting unit may be a hand-held electronic device, the patient interface device may include a visual display device, and/or the input device may include a key pad comprising a number of manually activated buttons. Alternatively or additionally, the input device may include a microphone. In this case, the processor may be configured to process voice messages received via the microphone and convert the voice messages to patient information. Alternatively, the processor may be configured to record the voice messages by storing them in memory. The stored voice messages may also be time and date stamped by the processor. In any case, the processor of the information collecting unit may be configured to store the patient information in the memory device. The system may further comprise an electronic device including a processor electrically coupled to a memory device, and means for transferring the patient information from the memory device of the information collecting unit to the memory device of the electronic device. In cases where the system further including a glucose measuring device configured to measure glucose concentration of a body fluid of the patient, and wherein the patient information further includes measurements of the patient's glucose concentration via the glucose measuring device, and information relating to timing and quantities of insulin delivered to the patient's body, the glucose measuring device may be separate from the information collecting unit. Alternatively, the glucose measuring device may be carried by the information collecting unit and electrically connected to the processor, and the glucose concentration measurements may be provided directly to the processor by the glucose measuring device.

In an alternative embodiment, the system may further comprising an electronic device separate from the information collecting unit, and the electronic device may include the patient interface device and the input device. Means may also be included for transferring the instructions from the information collecting device to the electronic device and for transferring the patient information from the electronic device to the information collecting device. In embodiments that include a glucose measuring device configured to measure glucose concentration of a body fluid of the patient, the glucose measuring device may be separate from the electronic device. Alternatively, the glucose measuring device may be carried by the electronic device. In any case, the electronic device may be a hand-held electronic device. The patient interface device may include a visual display device. The input device may include a key pad comprising a number of manually activated buttons. The processor of the information collecting unit may be configured to store the patient information in the memory device. The electronic device may be or include a cellular telephone. In this case, the patient interface device may include a speaker, the input device may include a key pad comprising a number of manually activated buttons, and/or the input device may include a microphone configured to receive voice messages from the patient.

A system for collecting patient information from which diabetes therapy may be determined may comprise a patient interface device, a patient notification device, an input device for entering patient information, and an information collecting unit. The information collecting unit may include a processor electrically coupled to a memory having stored therein at least one algorithm executable by the processor to activate the patient notification device followed by presenting instructions to the patient via the patient interface device for collecting specified information from the patient via the input device.

The patient interface device, the patient notification device and the input device may illustratively each be carried by the information collecting unit and may each be electrically connected to the processor. The information collecting unit may be a hand-held electronic device. The patient interface device may be or include a visual display device. The input device may be or include a key pad comprising a number of manually activated buttons. Alternatively or additionally, the input device may be or include a microphone, and the processor may be configured to process voice messages received via the microphone and convert the voice messages to patient information. Alternatively, the processor may be configured to record the voice messages by storing them in memory. The stored voice messages may also be time and date stamped by the processor. In any case, the patient notification device may be or include at least one of an audible, visual and a vibratory device. The processor of the information collecting unit may, in this embodiment, be configured to store the patient information in the memory device. The patient information may include, but should not be limited to, information relating to timing and carbohydrate amount of food consumed by the patient, the composition of food consumed by the patient, the patient's glucose concentration, insulin received by the patient, therapy related medication being taken by the patient and/or the physical state of the patient. The system may further comprise an electronic device including a processor electrically coupled to a memory device, and means for transferring the patient information from the memory device of the information collecting unit to the memory device of the electronic device.

In an alternative embodiment, the system may further comprise an electronic device separate from the information collecting unit. In this embodiment, the electronic device may include the patient interface device, the patient notification device and the input device. The system may further include means for transferring the instructions from the information collecting device to the electronic device and for transferring the patient information from the electronic device to the information collecting device. Illustratively, the electronic device may be a hand-held electronic device. The patient interface device may be or include a visual display device. The input device may be or include a key pad comprising a number of manually activated buttons. Alternatively or additionally, the input device may be or include a microphone configured to receive voice messages from the patient. In any case, the patient notification device may be or include at least one of an audible, visual and a vibratory device. The processor of the information collecting unit, in this embodiment, may be configured to store the patient information in the memory device. The patient information may include, but should not be limited to, information relating to timing and carbohydrate amount of food consumed by the patient, the composition of food consumed by the patient, the patient's glucose concentration, insulin received by the patient, therapy related medication being taken by the patient and/or the physical state of the patient. Alternatively, the electronic device may be or include a cellular telephone. In this case, the patient interface device may be or include a speaker, the input device may be or includes a key pad comprising a number of manually activated buttons, and/or the input device may be or include a microphone configured to receive voice messages from the patient.

In still another alternative embodiment, the system may further comprise an electronic device separate from the information collecting unit, wherein the electronic device may include the patient interface device and the input device, and means for transferring the instructions from the information collecting device to the electronic device and for transferring the patient information from the electronic device to the information collecting device. In this embodiment, the electronic device may be a telephone. The telephone may be a cellular telephone. The patient interface device may be or include a speaker. Alternatively or additionally, the patient interface device may be or include a visual display configured to reproduce text messages received by the telephone. The input device may be or include a key pad comprising a number of manually activated buttons. Alternatively or additionally, the input device may be or include a microphone configured to receive voice or text messages from the patient. The telephone may include an audible device that is activated by the telephone to provide notification of an incoming call. In this case, the information collection unit may further include means for communicating via a telephone network, wherein the patient notification device may be the audible device, and wherein the processor of the information collection unit may be configured to activate the patient notification device by placing a call to the telephone. Alternatively or additionally, the patient notification device may be or include a pager configured to be worn or carried by the patient. In this case, the information collection unit may further includes means for activating the pager via a paging network, and wherein the processor of the information collection unit may be configured to activate the patient notification device by activating the pager via the paging network. The pager may be configured to produce either of an audible signal and a vibratory signal when activated.

A system for collecting patient information from which diabetes therapy may be determined may comprise a patient interface device, an input device for modifying patient information, and an information collecting unit. The information collecting unit may include a processor electrically coupled to a memory having stored therein at least one algorithm executable by the processor to present default values of at least some of the patient information to the patient via the patient interface device, followed by presenting instructions to the patient via the patient interface device to replace any of the default values with corresponding actual values of patient information via the input device. In any case, the patient information may be stored in the memory of the information collecting unit. The patient information may include, but should not be limited to, information relating to timing and carbohydrate amount of food consumed by the patient, the composition of food consumed by the patient, the patient's glucose concentration, insulin received by the patient, therapy related medication being taken by the patient and/or the physical state of the patient.

A system for collecting patient information from which diabetes therapy may be determined may comprise a patient interface device, a patient notification device, an input device for entering patient information, and an information collecting unit. The information collecting unit may include a processor electrically coupled to a memory having stored therein at least one algorithm executable by the processor to activate the patient notification device followed by presenting a list of patient information records for the current day to the patient via the patient interface device, followed by presenting instructions to the patient via the patient interface device to correct information in any of the listed patient information records via the input device. The patient information records may be stored in the memory of the information collecting unit. The patient information records may include, but should not be limited to, information relating to timing and carbohydrate amount of food consumed by the patient, the composition of food consumed by the patient, the patient's glucose concentration, insulin received by the patient, therapy related medication being taken by the patient, and/or the physical state of the patient.

A method for collecting patient information from which diabetes therapy may be determined may comprise programming an information collecting device with instructions specific to a patient for entering patient information therein. The method may further comprise entering the patient information into the information collecting device by the patient according to the instructions, the patient information including information relating to timing and carbohydrate amount of food consumed by the patient and insulin received by the patient. The method may further comprise using at least some of the patient information entered into the information collecting device to determine a diabetes therapy for the patient.

A method for collecting patient information from which diabetes therapy may be determined may comprise programming an information collecting device with instructions specific to a patient for entering specified patient information therein. The method may further comprise activating a patient notification device at one or more predetermined times to alert the patient to enter the specified patient information into the information collecting device according to the instructions. The method may further comprise using at least some of the patient information entered into the information collecting device to determine a diabetes therapy for the patient.

A method for collecting patient information from which diabetes therapy may be determined may comprise programming an information collecting device with instructions specific to a patient for entering specified patient information therein. The method may further comprise programming the information collecting device with default values of the specified patient information. The method may further comprise presenting at least some of the default values to the patient. The method may further comprise prompting the patient, according to the instructions, to accept the default values if they represent actual values of the patient information and to otherwise replace any of the default values with corresponding actual values of patient information.

A method for collecting patient information from which diabetes therapy may be determined may comprise programming an information collecting device with instructions for presenting a list of existing patient information records to a patient. The method may further comprise executing the instructions to present the list of existing patient information records for the current day to the patient. The method may further comprise prompting the patient to modify information in any of the presented patient information records.

### BRIEF DESCRIPTION OF THE DRAWINGS

FIG. 1 is a block diagram representation of one illustrative embodiment of a system for collecting patient information from which diabetes therapy may be determined.
FIG. 2 is a block diagram representation of one illustrative embodiment of the patient glucose measuring device depicted in FIG. 1.
FIG. 3 is a block diagram representation of another illustrative embodiment of the patient glucose measuring device depicted in FIG. 1.
FIG. 4 is a block diagram representation of another illustrative embodiment of a system for collecting patient information from which diabetes therapy may be determined.
FIG. 5 is a flowchart of one illustrative process for collecting patient information from which diabetes therapy may be determined, using either of the systems of FIGS. 1 and 4.
FIG. 6 is a flowchart of one illustrative embodiment of sub-process A depicted in FIG. 5.
FIG. 7 is a flowchart of one illustrative embodiment of sub-process B depicted in FIG. 5.
FIG. 8 is a flowchart of one illustrative embodiment of sub-process C depicted in FIG. 5.
FIG. 9 is a flowchart of one illustrative embodiment of sub-process D depicted in FIG. 5.
FIG. 10 is a flowchart of one illustrative embodiment of sub-process E depicted in FIG. 5.
FIG. 11 is a flowchart of one illustrative embodiment of sub-process F depicted in FIG. 5.
FIG. 12 is a flowchart of an alternate or additional illustrative process for collecting patient information from which diabetes therapy may be determined, using either of the systems of FIGS. 1 and 4.
FIG. 13 is a flowchart of one illustrative embodiment of sub-process G depicted in FIG. 12.
FIG. 14 is a flowchart of one illustrative embodiment of sub-process H depicted in FIG. 12.
FIG. 15 is a flowchart of one illustrative embodiment of sub-process I depicted in FIG. 12.
FIG. 16 is a flowchart of one illustrative embodiment of sub-process J depicted in FIG. 12.
FIG. 17 is a flowchart of another alternate or additional illustrative process for collecting patient information from which diabetes therapy may be determined, using either of the systems of FIGS. 1 and 4.
FIG. 18 is a flowchart of one illustrative embodiment of step 404 of the process illustrated in FIG. 17.
FIG. 19 is a flowchart of an alternate or additional illustrative embodiment of step 404 of the process illustrated in FIG. 17.
FIG. 20 is a flowchart of another alternate or additional illustrative embodiment of step 404 of the process illustrated in FIG. 17.
FIG. 21, is a flowchart of one illustrative embodiment of step 406 of the process illustrated in FIG. 17.
FIG. 22 is a flowchart of one illustrative embodiment of a process for providing a summary of patient lifestyle information to be collected during the next or current day.

### DESCRIPTION OF THE ILLUSTRATIVE EMBODIMENTS

For the purposes of promoting an understanding of the principles of the invention, reference will now be made to a number of illustrative embodiments shown in the attached drawings and specific language will be used to describe the same.

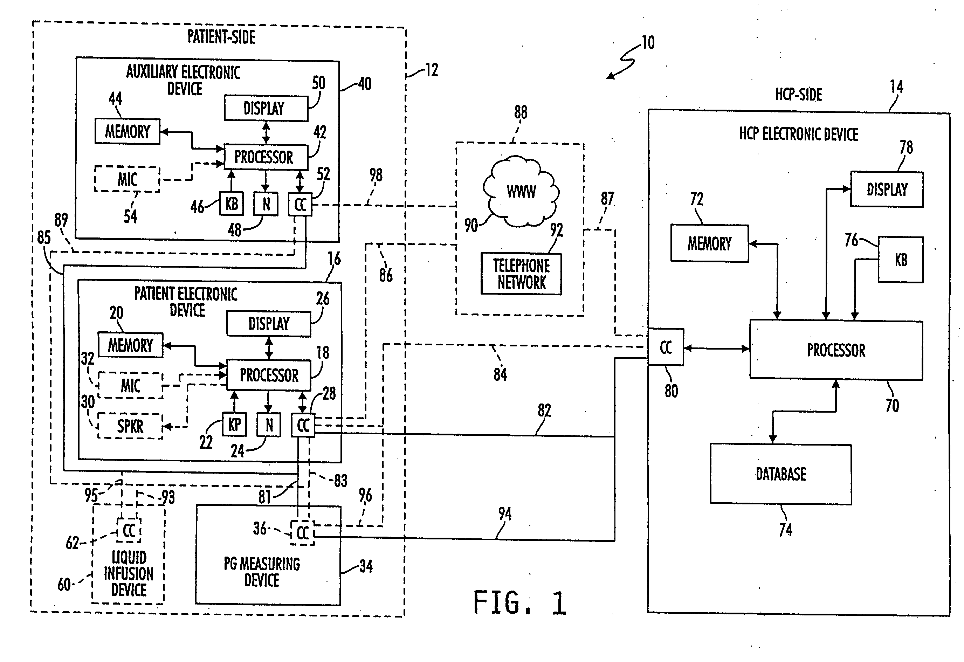

Referring to FIG. 1, a block diagram representation is shown of one illustrative embodiment of a system 10 for collecting patient information from which diabetes therapy may be determined. It will be understood that the system 10 may be used by a health care professional to establish or design an overall diabetes therapy or diabetes therapy schedule that will thereafter be followed by a patient, and/or to modify an existing overall diabetes therapy or diabetes therapy schedule that will thereafter be followed by the patient. In this context, the phrase "collecting patient information from which diabetes therapy may be determined" will accordingly be understood to mean collecting patient information from which an overall diabetes therapy or diabetes therapy schedule may be established or modified and which will then be thereafter followed by a patient. Examples of any such overall diabetes therapy or diabetes therapy schedule may include, but are not limited to, any one or more of insulin therapy or administration of any other blood glucose modifying drug, one or more other diabetes therapy related drugs, one or more dietary restrictions or specified dietary schedule, one or more specified meal times, one or more physical exercises or exercise schedules, one or more, and the like.

Generally, the system 10 includes one or more patient-side electronic devices 12 and at least one health care professional (HCP)-side electronic device 14. In the illustrated embodiment, for example, the patient-side devices 12 include a patient electronic device 16 having a processor 18 in data communication with a memory unit 20, a key pad (KP) 22, a notification device 24, a display device 26 and a communication circuit 28. The patient electronic device 16 may be provided in the form of a general purpose computer, personal computer (PC), laptop or notebook computer, a hand-held electronic device such as a personal data assistant (PDA), smart phone that may or may not include an on-board camera and that may or may not include instant messaging (e.g., SMS) capability, internet-accessible electronic device such as a BlackBerry® electronic communication device which may, but need not, include smart phone capabilities, or the like. The patient electronic device 16 may be configured to operate in accordance with one or more conventional operating systems including for example, but not limited to, Windows, Linux and Palm OS or the like.

The processor 18 is, in the illustrated embodiment, microprocessor-based, although the processor 18 may alternatively be formed of one or more general purpose and/or application specific circuits, and operable as described hereinafter. The memory unit 20 includes, in the illustrated embodiment, sufficient capacity to store data and one or more software algorithms executable by the processor 18. In some embodiments, as will be described hereinafter, the memory unit 20 may include a database in which collected patient information is stored temporarily or long term. The memory unit 20 may include one more conventional memory or other data storage devices.

The key pad 22 is conventional and may include, for example, a number of user-actuated buttons that may be manipulated in a conventional manner to input and/or modify data. The notification device 24 is a conventional notification device responsive to an activation signal provided by a processor 18 to produce visual, audible and/or vibratory signal or pattern of signals, or in the case of an audible device, one or more recorded or synthesized voice messages, music or the like. In the illustrated embodiment, the display 26 is a conventional display device including for example, but not limited to, a light emitting diode (LED) display, a liquid crystal display (LCD), a cathode ray tube (CRT) display, or the like, and may include one or more touch-responsive regions for receiving patient input of data. The communication circuit 28 may be or include a conventional data port configured for hard wire connection to another electronic device or system. Alternatively or additionally, the communication circuit 28 may be or include conventional electronic circuitry configured to communicate wirelessly with another electronic device or system via any conventional wireless communication technique and protocol including for example, but not limited to, inductive coupling, infrared (IR), radiofrequency (RF), Blue Tooth, WiFi, land-line telephone, cellular telephone, satellite telephone, internet, intranet, or the like. The patient electronic device 16 may further, in some embodiments, include one or more additional electronic components. For example, the patient electronic device 16 may include a speaker 30 or similar device configured to communicate audible information in the form of voice communication, one or more coded patterns, vibrations, synthesized voice messages or the like. The patient electronic device 16 may alternatively or additionally include a microphone 32 configured to receive voice messages from the patient, and to transfer corresponding signals to the processor 18 for further processing. Alternatively, the processor 18 may be configured to record the voice messages by storing them in memory. The stored voice messages may also be time and date stamped by the processor 18.

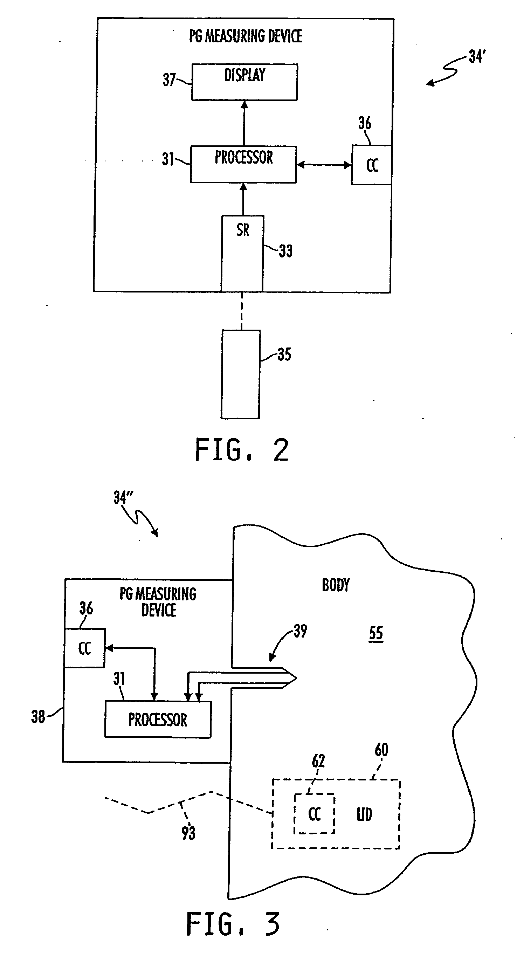

The patient-side electronic devices 12 further include a patient glucose measuring device 34 that is configured to measure the glucose concentration of a bodily fluid of the patient. The patient glucose measuring device 34 may further include a conventional communication circuit 36 identically as described hereinabove with respect to the communication circuit 28. Referring now to FIG. 2, a block diagram representation of one illustrative embodiment 34' of the patient glucose measuring device 34 of FIG. 1 is shown. In the illustrated embodiment, the patient glucose measuring device 34' is provided in the form of a conventional blood glucose measuring device having a processor 31 electrically connected to a strip reader 33, to a conventional display 37 and also to the communication circuit 36. The patient glucose measuring device 34' is operable in a conventional manner to receive a strip 35, upon which the patient has deposited a blood sample, in the strip reader 33. The processor 31 is configured in a conventional manner to process signals produced by the strip reader 33, and to display on the display unit 37 a value corresponding to the glucose concentration of the patient's blood deposited on the strip 35. The processor 31 may alternatively or additionally be configured to provide a signal, corresponding to the glucose concentration, to the communication circuit 36, and the communication circuit 36 may be configured to automatically transfer the glucose concentration value to another electronic device or system, via a hardwire or wireless interface, in a conventional manner.

Referring now to FIG. 3, a block diagram representation of another illustrative embodiment 34" of the patient glucose measuring device 34 of FIG. 1 is shown. In the illustrated embodiment, the device 34" is provided in the form of the glucose sensor module 38 attached to or integral with a glucose sensor portion 39 that is configured to be percutaneously inserted into the body 55 of a patient. The glucose sensor portion 39 typically has one or more sensor electrodes configured to sense the glucose concentration of the patient's body fluid, and to provide corresponding signals to the processor 31. The processor 31 is configured to process the signals produced by the sensor portion 39 and determine a corresponding glucose concentration value. The processor 31, in one illustrative embodiment, may be configured to provide a continuous signal corresponding to the glucose concentration. The communication circuit 36 may, in this embodiment, be configured to automatically transfer the glucose concentration value to another electronic device or system, via a hardwire or wireless interface, in a conventional manner. Alternatively, the device 34" may be provided in the form of an on-demand device requiring the patient to manually prompt the device 34" to transfer a glucose concentration value to another electronic device or system. In one specific embodiment, for example, the device 34" may include a switch or button (not shown), and the processor 31 may be responsive to manual activation of the switch or button to process the signals produced by the sensor portion 39, determine a glucose concentration value therefrom and transfer the glucose concentration value to another electronic device or system. In another specific embodiment, for example, the device 34" and the other electronic device or system, such as the patient electronic device 16, may each include proximity activated circuitry (not shown) that automatically establishes a communication link between the devices when brought into proximity with each other. In this embodiment, the processor 31 may be responsive to establishment of the communication link to automatically determine a glucose concentration value from the signals produced by the sensor portion 39 and to transfer the glucose concentration value to the other electronic device or system, e.g., the patient electronic device 16. Although not illustrated in FIG. 3, the glucose sensor module 38 may include a display, and the processor 31 may be configured in a conventional manner to display thereon a value corresponding to the glucose concentration of the patient.

Returning again to FIG. 1, the patient glucose measuring device 34 may illustratively be provided in the form of the strip reading device 34' shown in FIG. 2. In this embodiment, glucose concentration information may be provided to the processor 18 of the patient electronic device 16 in any of several ways. For example, the glucose concentration values may be read from the display 37 from the patient glucose measuring device 34', and manually provided to the processor 18 via the key pad 22. Alternatively, a hard wire connection 81 may be established between the communication circuit 28 of the patient electronic device 16 and the patient glucose measuring device 34', in which case the glucose concentration information may be automatically transferred from the glucose measuring device 34' to the processor 18 of the patient electronic device 16. Alternatively still, a wireless communication link 83 may be established between the communication circuit 28 of the patient electronic device 16 and the patient glucose measuring device 34', in which case the glucose concentration information may automatically transferred from the patient glucose measuring device 34' to the processor 18 of the patient electronic device 16 via the wireless communication link 83. Alternatively still, the patient glucose measuring device 34' of FIG. 2 may be incorporated into, and therefore carried by, the patient electronic device 16. In this embodiment, the patient electronic device 16 includes a strip reader 33 that is electrically connected to the processor 18 of the patient electronic device 16, so that glucose concentration information determined by the strip reader 33 is provided directly to the processor 18.

The patient glucose measuring device 34 of FIG. 1 may alternatively be provided in the form of the patient glucose sensor module 34" illustrated in FIG. 3. In this embodiment, glucose concentration measurements are transferred from the processor 31 of the patient glucose sensor module 34" to the processor 18 of the patient electronic device 16 through the hard wire communication link 81 or the wireless communication link 83.

The patient-side electronic devices 12 may further include an auxiliary electronic device 40 including a processor 42 electrically connected to a conventional memory unit 44, a conventional keyboard (KB) 46, a conventional notification device 48, a conventional display unit 50 and a conventional communication circuit 52. The auxiliary electronic device 40 may further include a microphone 54 that is electrically connected to the processor 42. The auxiliary electronic device 40 may be provided in the form of a general purpose computer, personal computer (PC), laptop or notebook computer, or the like, and the components 42-52 are conventional components typically provided with such a device 40. The auxiliary electronic device 40 may illustratively be used to download data stored in the patient electronic device 16 for the purpose of storing such data in the memory 44 and/or for the purpose of transferring such data to another electronic device or system. In this regard, a conventional hard wired communication path 85 and/or a conventional wireless communication path 89 may be established between the communication circuit 28 of the patient electronic device 16 and the communication circuit 52 of the auxiliary electronic device 40.

In some embodiments, the patient may have an implanted or externally worn infusion device 60 that is configured to deliver a glucose-lowering drug, e.g., insulin, to the patient in a conventional manner. In such cases, the liquid infusion device 60 may include a conventional communication circuit 62 that may be configured for a hard-wired connection 95 with the communication circuit 28 of the patient electronic device 16, the communication circuit 36 of the patient glucose measuring device 34 and/or the communication circuit 52 of the auxiliary electronic device 40, and/or that may be configured to establish a wireless communication link 93 between the communication circuit 62 and any such communication circuits 28, 36 and/or 52. In embodiments wherein the liquid infusion device 60 is an externally-worn infusion device, the communication circuit 52 may be configured for hard wire communications and/or wireless communications with any of the devices 16, 34 and 40. In other embodiments wherein the liquid infusion device 60 is an implanted infusion device, communications between the device 60 and any of the devices 16, 34 and 40 are generally carried out via the wireless communication link 93, as illustrated in dashed-line representation in FIG. 3. In either case, liquid infusion information, e.g., insulin delivery information, may be automatically transferred from the liquid infusion device 60 to the patient glucose measuring device 34, to the processor 18 of the patient electronic device 16 and/or to the processor 42 of the auxiliary electronic device 40 via the hard wire link 95 and/or wireless link 93. As used herein, the term "insulin delivery information" includes any information relating to delivery of insulin to the patient including, for example, but not limited to, insulin delivery type, e.g., basal, correction bolus or meal compensation bolus as these terms are generally understood in the art, insulin quantity or amount (e.g., in international units or I.U.), insulin delivery pattern, e.g., single or multiple delivery events, and insulin delivery rates (e.g., speed of delivery of the one or more insulin delivery events). In embodiments that do not include a liquid infusion device 60 and insulin or another blood glucose lowering drug is instead delivered to the patient via manual injection or other manual administering technique, the insulin delivery information may alternatively be manually provided to the patient electronic device 16 via the key pad 22 and/or microphone 32, and may alternatively or additionally be manually provided to the auxiliary electronic device 40 via the keyboard 46 or microphone 54.

In the embodiment illustrated in FIG. 1, the health care professional (HCP) side devices include a single electronic device 14 having a processor 70 that is electrically connected to a memory unit 72, a database 74, a keyboard 76, a display united 78 and a communication circuit 80. The electronic device 14 may be provided in the form of a general purpose computer, central server, personal computer (PC), laptop or notebook computer or the like. The electronic device 14 may be configured to operate in accordance with one or more conventional operating systems including for example, but not limited to, Windows, Linux, Palm, etc., and may also be configured to process data according to one or more conventional internet or telephone communication protocols. The processor 70 is, in the illustrated embodiment, microprocessor-based, although the processor 70 may alternatively be formed of one or more general purpose and/or application specific circuits and operable as described hereinafter.

The memory unit 72 includes, in the illustrated embodiment, sufficient capacity to store data and one or more software algorithms executable by the processor 70. The database 74 is a conventional database configured to store patient information. The database 74 may include the memory of 72 or the memory unit 72 may include the database 74.

The keyboard 76 is a conventional keyboard and may be used in a conventional manner to input and/or modify data. The display unit 78 is likewise a conventional display unit that may be controlled by the processor 70 to display information in the form of text, icons, graphical images, photographs, video sequences and the like.

The communication circuit 80 may be configured for hard wire or wireless communication. In the embodiment illustrated in FIG. 1, for example, a conventional hard wire connection 82 may be established between the communication circuit 28 of the patient electronic device 16 and the communication circuit 80 of the electronic device 14. Alternatively or additionally, a conventional hard wire connection 94 may be established between the communication circuit 36, in embodiments of the patient glucose measuring 34 that include the communication circuit 36, and the communication circuit 80 of the electronic device 14. Alternatively or additionally, a conventional local wireless communication link 84 may be established between the communication circuit 28 of the patient electronic device 16 and the communication circuit 80 of the electronic device 14, and/or a conventional local wireless communication link 96 may be established between the communication circuit 36, in embodiments of the patient glucose measuring device 34 that include the communication circuit 36, and the communication circuit 80 of the electronic device 14. Alternatively or additionally, a conventional long-distance wireless communication medium 88 may be established between the patient electronic device 16 and the electronic device 14, and/or between the auxiliary electronic 40 and the electronic device 14. For example, a conventional wireless telephone link 86 may be established between the communication circuit 28 of the patient electronic device 16 and a cellular or satellite telephone network 92, and a similar wireless communication link 87 may be established between the communication circuit 80 of the electronic device 14 and the wireless or satellite telephone network 92. Alternatively, the telephone network 92 may represent a conventional land-line telephone network, and a land-line telephone connection may be established between the electronic device 14 and the patient electronic device 16 and/or the auxiliary electronic device 40. Alternatively or additionally, the communication links 86 and 87 may represent internet links to the world-wide web (WWW) 90. Similar telephone or internet links 98 and 87 may be established between the communication circuit 52 of the auxiliary electronic device 40 and the communication circuit 80 of the electronic device 14. In one exemplary embodiment, only one of the patient-side device(s) 12 or the HCP electronic device(s) 14 may control the content one or more web sites that may be accessed, e.g., viewed, via the WWW by the other party. In an alternatively embodiment, both of the patient-side device(s) 12 or the HCP electronic device(s) 14 may access and post information to the one or more web sites.

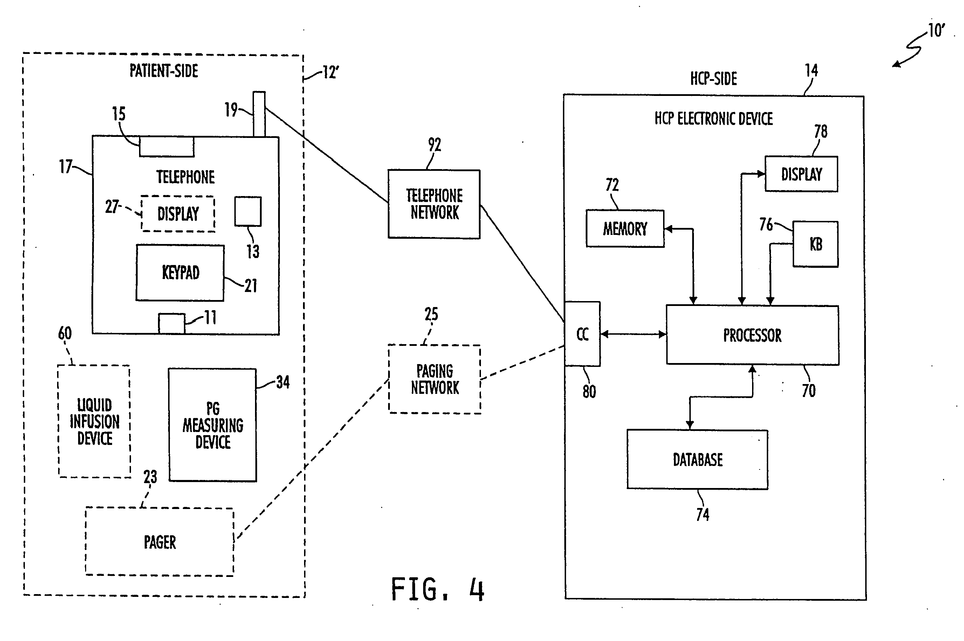

Referring now to FIG. 4, a block diagram representation of another illustrative embodiment of a system 10' is shown for collecting patient information from which diabetes therapy may be determined. In the illustrated embodiment, the health care professional-side device 14 is identical to that described hereinabove with respect to FIG. 1. The patient-side devices 12' include a conventional telephone 17 including conventional telephone components. Examples of such conventional telephone components include, but are not limited to, a conventional key pad 21, a conventional microphone or transducer 11, a conventional speaker or earpiece 15, a conventional incoming call alerting device 13 and a conventional antenna 19. The incoming call alerting device 13 may be or include any one or more of a conventional electromechanical ringer device, an audible electronic device configured to emit a pattern of tones, recorded or synthesized music or voice message, a visual indicator, a tactile indicator such as a vibration device, or the like. The telephone 17 may illustratively be a conventional land-line telephone or a wireless telephone configured for cellular, satellite or VOIP (voice over internet protocol) communications. The telephone 17 may, in some embodiments, further include a display 27 as shown in dashed-line form in FIG. 4. In any case, telephone contact between the telephone 17 and the communication circuit 80 of the electronic device 14 is accomplished in a conventional manner via a telephone network 92.

The patient-side 12' may further include a patient glucose measuring device 34, as described hereinabove, and may also optionally include a liquid infusion device 60 of the type described hereinabove. The patient-side 12' may further include a conventional pager 23, and the communication circuit 80 of the electronic device 14 may be configured to contact the pager 23 via conventional paging network 25. The pager 23 is responsive to contact by the electronic device 14 to emit a tone, pattern of tones and/or vibrate in a conventional manner.

The system 10 of FIG. 1 or the system 10' of FIG. 4 may be used to collect patient information from which diabetes therapy may be determined. With either system 10 or 10', at least one of the devices of the system is programmed to provide for patient input of specific lifestyle information, typically in real-time, pseudo-real-time or on a periodic, e.g., daily, basis. The type and amount of lifestyle information to be entered into the system 10 or 10' will typically be patient-specific and will be determined by a health care professional on a patient-by-patient basis. Patient compliance in entering such information into the system 10 and 10' is expected to vary from patient-to-patient, and the programming of the at least one device of the system will therefore typically be also carried out on a patient-by-patient basis to provide information collection instructions to the patient in a manner the results in a desired amount or degree of patient guidance and autonomy throughout a specified information collection time period, which may typically range from a few days to several weeks or months. For example, some patients will be adept at timely and consistently entering useful lifestyle information into the system 10 or 10', and for these patients the programming may be carried out in a manner that allows the patient to initiate entry of information into the system 10 or 10' and to choose the type of information to be entered. Conversely, other patients will require more guidance and/or reminders of when to enter information, and for these patients the programming may be carried out in a manner that notifies the patient whenever at least certain types of information should be entered into the system 10 or 10', and/or guides the patient through specific information collection scenarios. In any case, the programming of the at least one device of the system results in presenting to the patient of one or more instructions for entering patient information.

The system 10 or 10' may be implemented in many different forms, and a number of examples will now be given of specific implementations of the system 10 or 10' illustrated and described herein. It will be understood that these examples are provided to illustrate some, but not all, possible structural implementations of the system 10 or 10', and that these examples should therefore not limit the system 10 or 10' to these example implementations. Any specific implementation of the system 10 or 10' will include a patient interface for presenting instructions to the patient, an input device configured to receive input of patient information and an information collecting unit having a processor electrically coupled to a memory having stored therein at least one algorithm executable by the processor to present instructions to the patient via the patient interface device for collecting the patient information from the patient via the input device. Any specific implementation of the system 10 or 10' may further include a patient notification device which may be activated by the processor pursuant to the one or more algorithms to notify the patient of a particular event. In each of the following examples of the system 10 or 10', the structural feature or features that correspond to the patient interface, information collecting device, input device and the notification device will be identified.

In one example implementation of the system 10 of FIG. 1, the patient electronic device 16 is the information collecting unit and is provided in the form of a personal data assistant (PDA) or other handheld electronic device. In this implementation, the memory 20 has stored therein at least one algorithm executable by the processor 18 to present instructions to the patient via the patient interface device for collecting the patient information from the patient via the input device. The patient interface in this implementation may be or include the display 26, in which case the processor 18 is configured to display visual instructions to the patient, and/or the speaker 30, in which case the processor 18 is configured to present audible instructions to the patient. The input device in this implementation may be or include the keypad 22, in which case the patient manually enters at least a portion of the patient information in a conventional manner, the microphone 32, in which case the processor 18 is configured to receive and process or store voice information provided by the patient via the microphone 32, and/or the display 26 configured to include one or more touch-sensitive buttons, in which case the patient manually enters at least a portion of the patient information via such a touch-screen display. The notification device in this implementation may be or include the notification device 24 described hereinabove, the display 26 and/or the speaker 30. The patient glucose measuring device 34 in this implementation may be provided in either of the forms illustrated in FIGS. 2 or 3, or may alternatively be incorporated into the patient electronic device 16 as described hereinabove. Patient glucose information may be manually entered into the patient electronic device 16 or may alternatively be transferred automatically from the patient glucose measuring device 34 to the processor 18 as described hereinabove. Insulin delivery information may likewise be manually or automatically transferred to the processor 18 via the liquid infusion device 60 in embodiments that include the liquid infusion device 60. Alternatively still, patient glucose information and/or insulin delivery information may be transferred directly from the patient glucose measuring device 34 to the HCP electronic device 14 via the hard wire interface 94 or the wireless interface 96.

In this implementation, the patient electronic device 16 is programmed, e.g., by a health care professional, with one or more algorithms that are executable by the processor 18 to guide a patient through a patient information collection time period. Examples of various embodiments of the one or more algorithms will be provided and described in detail hereinafter. Initially, the patient information entered into the patient electronic device 16 is stored in the memory 20. The patient information may be subsequently transferred to the HCP electronic device 14 for storage in the database 74 via the hard wire interface 82 or the wireless interface 84. The auxiliary electronic device 40 may optionally be provided in this implementation as a data backup device and/or as a device for transferring the collection of patient information to the HCP electronic device 14. In such cases, the patient information may be transferred from the patient electronic device 16 to the auxiliary electronic device 40 via the hard wire interface 85 or the wireless interface 89. The patient information may then be transferred from the auxiliary electronic device 40 to the HCP electronic device 14 via the wireless interfaces 98 and 87, e.g., via the WWW 90 or a telephone network 92. In any case, the health care professional may then access the patient information stored in the database 74 to analyze this data and design a diabetes therapy for the patient, or modify an existing diabetes therapy, that is based on this analysis.

In another example implementation of the system 10 of FIG. 1, the patient electronic device 16 is the information collecting unit and is provided in the form of a personal computer, laptop computer, notebook computer or the like. In this implementation, the memory 20 has stored therein at least one algorithm executable by the processor 18 to present instructions to the patient via the patient interface device for collecting the patient information from the patient via the input device. The patient interface in this implementation may be or include the display 26, in which case the processor 18 is configured to display visual instructions to the patient, and/or the speaker 30, in which case the processor 18 is configured to present audible instructions to the patient. The input device in this implementation may be or include the keypad 22, in which case the patient manually enters at least a portion of the patient information in a conventional manner via the keypad 22, the microphone 32, in which case the processor 18 is configured to receive and process or store voice information provided by the patient via the microphone 32, and/or conventional mouse or other point-and-click device (not shown), in which case the processor 18 is configured to receive at least a portion of the patient information via manual selection of information presented to the patient via the display 26. The notification device in this implementation may be or include the notification device 24 described hereinabove, the display 26 and/or the speaker 30. The patient glucose measuring device 34 in this implementation may be provided in either of the forms illustrated in FIGS. 2 or 3, or may alternatively be incorporated into the patient electronic device 16 as described hereinabove. Patient glucose information may be manually entered into the patient electronic device 16 or may alternatively be transferred automatically from the patient glucose measuring device 34 to the processor 18 as described hereinabove. Insulin delivery information may likewise be manually or automatically transferred to the processor 18 via the liquid infusion device 60 in embodiments that include the liquid infusion device 60. Alternatively still, patient glucose information and/or insulin delivery information may be transferred directly from the patient glucose measuring device 34 to the HCP electronic device 14 via the hard wire interface 94 or the wireless interface 96.

In this implementation, the patient electronic device 16 is programmed, e.g., by a health care professional, with one or more algorithms that are executable by the processor 18 to guide a patient through a patient information collection time period. Examples of various embodiments of the one or more algorithms will be provided and described in detail hereinafter. Initially, the patient information entered into the patient electronic device 16 is stored in the memory 20. The patient information may be subsequently transferred to the HCP electronic device 14 for storage in the database 74 via the hard wire interface 82 or the wireless interface 84. The auxiliary electronic device 40 typically would be omitted in this implementation, but may optionally be included as a data backup device. In such cases, the patient information may be transferred from the patient electronic device 16 to the auxiliary electronic device 40 via the hard wire interface 85 or the wireless interface 89. In any case, the health care professional may access the patient information stored in the database 74 to analyze this data and design a diabetes therapy for the patient, or modify an existing diabetes therapy, that is based on this analysis.

In still another example implementation of the system 10 of FIG. 1, the HCP electronic device 14 is the information collecting unit, and in this implementation the memory 72 has stored therein at least one algorithm executable by the processor 70 to present instructions to the patient via the patient interface device for collecting the patient information from the patient via the input device. The patient interface in this implementation is the patient electronic device 16 generally, which may be provided in the form of a hand held electronic device, personal computer, laptop computer or notebook computer, in which case instructions may be conveyed to the patient in visual form, in which case the processor 70 is configured to display visual instructions to the patient via the display 26, and/or in audible form, in which case the processor 70 is configured to present audible instructions to the patient via the speaker 30. The input device in this implementation may be or include the keypad 22, in which case the patient manually enters at least a portion of the patient information in a conventional manner via the keypad 22, the microphone 32, in which case the processor 18 is configured to receive and process or store voice information provided by the patient via the microphone 32, the display 26 configured to include one or more touch-sensitive buttons, in which case the patient manually enters at least a portion of the patient information via such a touch-screen display, and/or a conventional mouse or other point-and-click device (not shown), in which case the processor 18 is configured to receive at least a portion of the patient information via manual selection of information presented to the patient via the display 26. The notification device in this implementation may be or include the notification device 24 described hereinabove, the display 26 and/or the speaker 30. The patient glucose measuring device 34 in this implementation may be provided in either of the forms illustrated in FIGS. 2 or 3, or may alternatively be incorporated into the patient electronic device 16 as described hereinabove. Patient glucose information may be manually transferred to the HCP electronic device 14 via the patient electronic device 16 or may alternatively be transferred automatically from the patient glucose measuring device 34 to the processor 18 as described hereinabove. Alternatively still, patient glucose information and/or insulin delivery information may be transferred directly from the patient glucose measuring device 34 to the HCP electronic device 14 via the wireless interface 96.

In this implementation, the HCP electronic device 14 is programmed, e.g., by a health care professional, with one or more algorithms that are executable by the processor 70 to guide a patient through a patient information collection time period. Examples of various embodiments of the one or more algorithms will be provided and described in detail hereinafter. In this implementation, the communication link 86, 87 is first established between the patient electronic device 16, e.g., the patient interface, and the HCP electronic device 14. In one embodiment, initiation of the communication link 86, 87 is undertaken by the patient electronic device 16 pursuant to instructions by the by patient to the patient electronic device 16 to establish this link. In this embodiment, the patient thus initiates the entering of information into the HCP electronic device 14. Alternatively, initiation of the communication link 86, 87 is undertaken by the HCP electronic device 14 pursuant to the one or more algorithms being executed by the processor 70. In this embodiment, the HCP electronic device 14 thus initiates contact with the patient electronic device 16 to request information from the patient. Such a request by the HCP electronic device 14 will typically take the form of activation by the HCP electronic device 14 of the patient notification device. In either case, information is provided by the patient to the patient electronic device 16, which is then automatically transferred to the HCP electronic device 14 via the WWW 90 or telephone network 92. The patient information is then entered by the HCP electronic device 14 directly into the database 74. The health care professional may subsequently access and analyze this data, and design a diabetes therapy for the patient, or modify an existing diabetes therapy, that is based on this analysis. The auxiliary electronic device 40 typically would be omitted in this implementation.

In an example implementation of the system 10' of FIG. 4, the HCP electronic device 14 is the information collecting unit, and in this implementation the memory 72 has stored therein at least one algorithm executable by the processor 70 to present instructions to the patient via the patient interface device for collecting the patient information from the patient via the input device. The patient interface in this implementation is the telephone 17, which may be provided in the form of a conventional land-line telephone or a wireless telephone configured for cellular, satellite or VOIP (voice over internet protocol) communications. In embodiments wherein the telephone 17 includes a display 27, instructions may be conveyed to the patient in this implementation in visual form, in which case the processor 70 is configured to display visual instructions to the patient via the display 27. Instructions may alternatively or additionally be conveyed to the patient in audible form, in which case the processor 70 is configured to present audible instructions to the patient via the speaker 15. The input device in this implementation may be or include the keypad 21, in which case the patient manually enters at least a portion of the patient information in a conventional manner via the keypad 21 and/or the microphone 11, in which case the processor 70 is configured to receive and process or store voice information provided by the patient via the microphone 11. The notification device in this implementation may be or include the incoming call alerting device 13 described hereinabove, in which case the processor 70 is configured to initiate contact with the telephone 17 by activating the incoming call alerting device 13 to signal an incoming call to the telephone 17. When the patient answers the telephone 17 in a conventional manner, a communications link between the telephone 17 and the HCP electronic device 14 is thereby established. Alternatively or additionally, the notification device in this implementation may be or include the pager 23, in which case the processor 70 is configured to notify the patient by contacting the pager 23 via the paging network 25. When the pager 23 is thereafter activated as described above, the patient is alerted to a patient information collection event, in which case the patient places a call to a pre-established telephone number to thereby establish a communication link between the telephone 17 and the HCP electronic device 14. The patient glucose measuring device 34 in this implementation will typically be provided in the form illustrated in FIG. 2, or may alternatively be incorporated into the telephone 17, in which case the telephone 17 will typically include a strip reader electrically connected to conventional circuitry configured to process the strip and determine a corresponding patient glucose concentration value. The corresponding patient glucose value may then be communicated to the patient in visual form via the display 21 and/or in audible form via the speaker 15. Patient glucose information and insulin delivery information may be manually transferred to the HCP electronic device 14 via the keypad 21 or microphone 11. Alternatively, patient glucose information and/or insulin delivery information may be transferred directly from the patient glucose measuring device 34 to the HCP electronic device 14 via a wireless interface as described with respect to FIG. 1.

In this implementation, the HCP electronic device 14 is programmed, e.g., by a health care professional, with one or more algorithms that are executable by the processor 70 to guide a patient through a patient information collection time period. Examples of various embodiments of the one or more algorithms will be provided and described in detail hereinafter. In this implementation, a communication link is first established between the telephone 17, e.g., the patient interface, and the HCP electronic device 14 via the telephone network 92. In one embodiment, initiation of this communication link is undertaken by the patient by placing a call via the telephone 17 to a pre-established telephone number associated with the HCP electronic device 14. Alternatively, the HCP electronic device may initiate the communication link by placing a call to the telephone and/or by activating the pager 23 as described hereinabove. In either case, information is provided by the patient to the telephone 17, which is then automatically transferred to the HCP electronic device 14 via the telephone network 92 (or WWW in the case of VOIP communications). The patient information is then entered by the HCP electronic device 14 directly into the database 74. The health care professional may subsequently access and analyze this data, and design a diabetes therapy for the patient, or modify an existing diabetes therapy, that is based on this analysis.

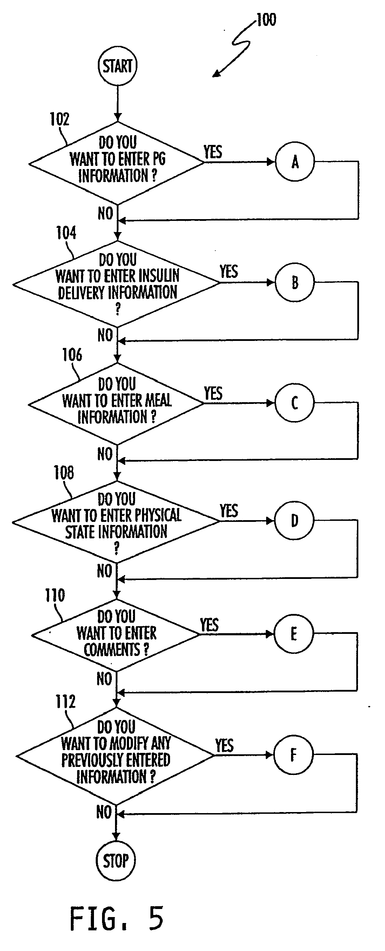

As described hereinabove, either the patient electronic device 16 or the HCP electronic device 14 is programmed to guide the patient, via one or more sets of instructions, through an information collection time period, which may typically range from a few days to several weeks or months. Referring now to FIG. 5, a flowchart is shown of one illustrative process 100 for collecting patient information from which diabetes therapy may be determined using either of the systems of FIGS. 1 and 2. In the illustrated embodiment, the process 100 is provided in the form of one or more software algorithms that may be executed by the processor 18 of the patient electronic device 16 or by the processor 70 of the electronic device 14 depending upon the specific implementation of the system 10 or 10' as described hereinabove. In any case, the process 100 begins at step 102 where the patient is asked via the patient interface whether the patient wants to enter patient glucose information into the information collecting unit. If so, the patient responds via the input device and execution of the process 100 advances to sub-process A. If the patient does not want to enter patient glucose information at step 102, the patient responds appropriately via the input device and the process 100 advances to step 104.

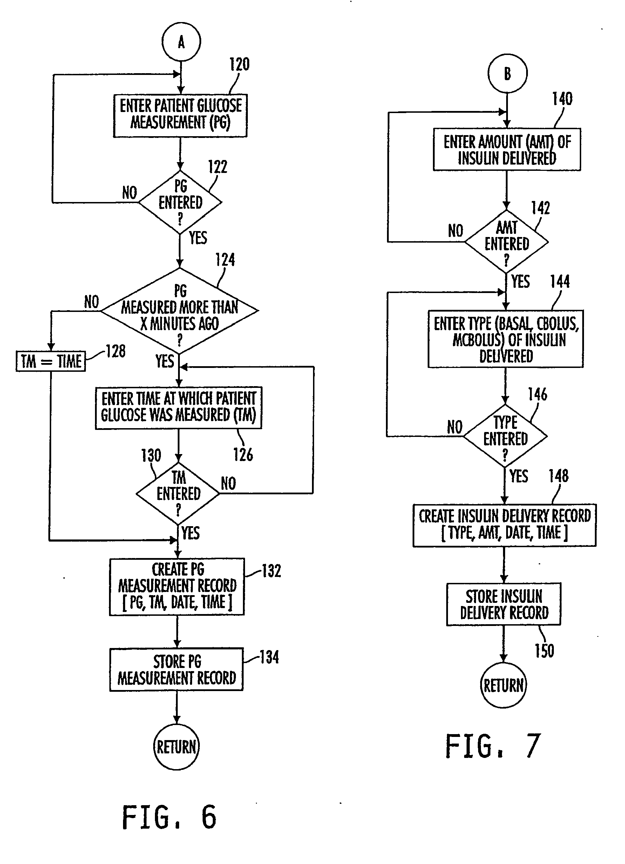

Referring now to FIG. 6, a flowchart is shown of one illustrative embodiment of sub-process A of FIG. 5. Sub-process A begins at step 120 where the processor of the patient collection unit instructs the patient via the patient interface to enter via the input device the patient glucose measurement (PG) taken by the patient glucose measurement device 34. Thereafter at step 122, information collecting unit processor determines whether PG has been entered. If not, the sub-process A loops back to step 120 until the patient enters the PG value. When the PG value has been entered, execution of the sub-process A advances to step 124 where the information collecting unit processor asks the patient via the patient interface device whether the patient glucose measurement entered at step 120 has been more than X minutes ago. If not, the sub-process A advances to step 128 where a time marker TM, is set equal to the current time. If, at step 124, the patient responds via the input device that the PG value entered at step 120 was measured more than X minutes ago, the sub-process A advances to step 126 where the information collecting unit processor instructs the patient to enter via the input device a time marker (TM), corresponding to the time at which the patient glucose value (PG) entered at step 120 was measured. Thereafter at step 130, the information collecting unit processor determines whether the time marker (TM) has been entered. If not, the sub-process A loops back to step 126 until the patient enters the time marker, TM. Following step 128, and following the "YES" branch of step 130, the sub-process A advances to step 132 where the information collecting unit processor creates a patient glucose measurement record. Illustratively, a PG measurement record take the form [PG, TM, DATE, TIME], where PG is the patient glucose measurement value, TM is the time marker corresponding to the time that the patient glucose value was measured, DATE is the current calendar date, and TIME is the current time. If the patient responds at step 124 that the patient glucose value was not measured more than X minutes ago, TM and TIME will have the same value. In any case, the sub-process A advances from step 132 to step 134 where the information collecting unit processor stores the PG measurement record in memory. Thereafter, the sub-process A is returned to its calling routine. It will be understood that in embodiments of the system 10 wherein the patient glucose measurements are automatically transferred from the patient glucose measuring device 34 to the patient electronic device 16, 102 of the process 100, as well as the sub-process A, are not necessary and may be omitted.

Returning again to FIG. 5, process 100 advances from the "NO" branch of step 102 and from the completion of sub-process A, to step 104 where the information collecting unit processor asks the patient via the patient interface device whether the patient wants to enter insulin delivery information. If so, the patient responds appropriately via the input device, and the process 100 advances to sub-process B. If the patient does not want to enter insulin delivery information, the patient responds appropriately via the input device at step 104, and the processor 100 advances to step 106.

Referring now to FIG. 7, a flowchart is shown of one illustrative embodiment of the sub-process B of FIG. 5. In the illustrated embodiment, sub-process B begins at step 140 where the information collecting unit processor instructs the patient via the patient interface to enter via the input device an amount, AMT, of insulin that was just delivered to the patient's body. Thereafter at step 142, the information collecting unit processor determines whether the insulin amount, AMT, has been entered into the information collecting unit by the patient. If not, the sub-process B loops back to step 140 until the patient enters the AMT of insulin just delivered to the patient's body. Following the "YES" branch of step 142, the process advances to step 144 where the information collecting unit processor instructs the patient to enter the type of insulin just delivered to the patient's body. The type of insulin delivered may be a basal insulin delivery, a correction bolus, CBOLUS, or a meal compensation bolus, MCBOLUS. Following step 144, the sub-process B advances to step 146 where the information collecting unit processor determines whether the patient has entered the insulin type at step 144. If not, the sub-process B loops back to step 144 until the patient enters or selects from a menu an appropriate type of insulin just delivered. Following the "YES" branch of step 146, the sub-process B advances to step 148 where the information collecting unit processor creates an insulin delivery record. Illustratively, the insulin delivery record may take the form [TYPE, AMT, DATE, TIME], where TYPE is the type of insulin just delivered, e.g., BASAL, CBOLUS, or MCBOLUS, AMT is the amount, e.g., in international units or I.U., of insulin just delivered, DATE is the current calendar date and TIME is the current time of day. Sub-process B advances from step 148 to step 150 where the information collecting unit processor stores the insulin delivery record in memory. Thereafter, the sub-process B is returned to its calling routine. It will be understood that the sub-process B illustrated in FIG. 7 may be modified to include more or less information relating to insulin delivery. Examples of additional information that the patient may be instructed to enter includes, but is not limited to, insulin delivery pattern (e.g., number and timing of multiple insulin deliveries) and insulin delivery rate (e.g., speed of insulin delivery in units such as amount/second). It will further be understood that in embodiments of the system 10 that include a liquid infusion device 60 that is configured to automatically transfer insulin delivery information to the patient electronic device 16, step 102 of the process 100 and sub-process B are unnecessary and may be omitted.

Returning again to FIG. 5, the process 100 advances from the "NO" branch of step 104, and from completion of the sub-process B, to step 106 where the information collecting unit processor asks the patient via the patient interface whether the patient wants to enter meal information. If so, the patient responds appropriately via the input device and execution of the process 100 advances to sub-process C. If the patient does not want to enter meal information, the patient responds appropriately via the input device at step 106, and the process 100 advances to step 108.

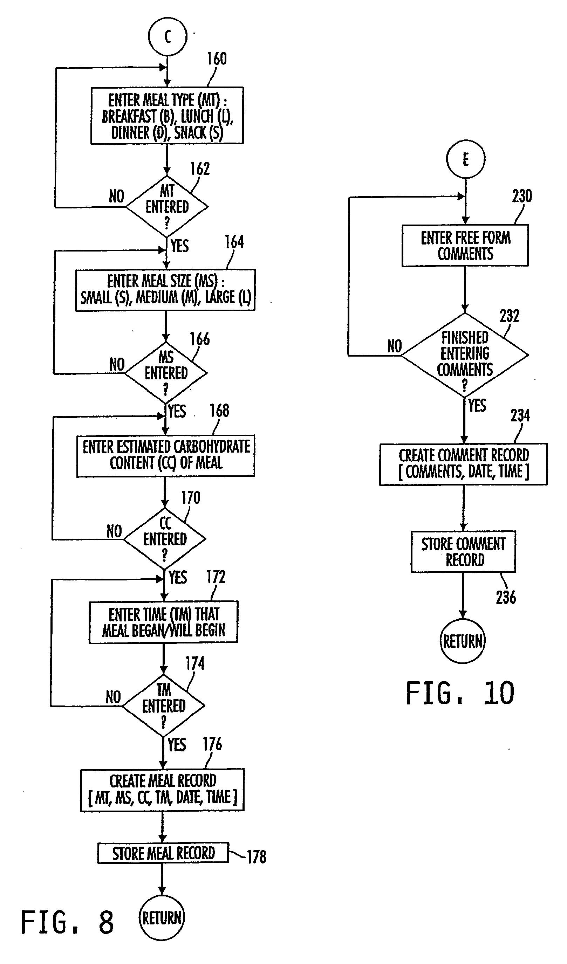

Referring now to FIG. 8, a flowchart of one illustrative embodiment of the sub-process C of FIG. 5 is shown. In the illustrated embodiment, the sub-process C begins at step 160 where the information collecting unit processor instructs the patient via the patient interface to enter a meal type, MT, via the input device. The patient may enter a meal type or be presented with a menu from which to choose the meal type, and in any case, the patient's choices will typically be breakfast, B, lunch, L, Dinner, D, and snack, S. The sub-process C thereafter advances to step 162 to determine whether the patient has entered MT. If not, the sub-process C loops back to step 160 until the patient enters the MT. From the "YES" branch of step 162, the sub-process C advances to step 164 where the information collecting unit processor instructs the patient via the patient interface to enter a meal size, MS, via the input device. The patient may enter a meal size or be presented with a menu from which to choose a meal size at step 164, and in any case, the patient's choices for meal size may be, for example, small, S, medium, M, and large, L. From step 164 the sub-process C advances to step 166 where the information collecting unit processor determines whether the patient has entered the meal size value. If not, the sub-process C loops back to step 164 until the patient enters the meal size value.

From the "YES" branch of 166, the sub-process C advances to step 168 where the information collecting unit processor instructs the patient via the patient interface to enter an estimated carbohydrate content, CC, of the meal via the input device. Thereafter at step 170, the information collecting unit processor determines whether the patient has entered the carbohydrate content value. If not, the sub-process C loops back to step 168 until the patient enters the estimated carbohydrate content value, CC. From the "YES" branch of step 170, the sub-process C advances to step 172 where the information collecting unit processor instructs the patient via the patient interface to enter the time, TM, that the meal began or will begin via the input device. Thereafter at step 174, the information collecting unit processor determines whether the patient has entered TM. If not, the sub-process C loops back to step 172 until the patient enters the time value, TM. From the "YES" branch of step 174, the sub-process C advances to step 176 where the information collecting unit processor creates a meal record. Illustratively, the meal record may take the form of [MT, MS, CC, TM, DATE, TIME] where MT is the meal type, e.g., B, L, D or S, MS is the meal speed, e.g., S, M, L, CC is the estimated carbohydrate content of the meal, TM is the time the meal began or will begin, DATE is the current calendar and TIME is the current time of day. From step 176, the sub-process C advances to step 178 where the information collecting unit processor stores the meal record in memory. From step 178, the sub-process C is returned to its calling routine. It will be understood that the sub-process C may be modified to require the patient to enter more or less meal-related information, and examples of additional meal information that may be required to be entered by the patient include, but are not limited to, a meal speed value, corresponding to the speed at which the meal is consumed, a total glycemic index of the meal, and meal size in terms of fat content, carbohydrate content and protein content. The term "glycemic index" is defined for purposes of this document as a parameter that ranks meals and snacks by the speed at which the meals or snacks cause the patient's blood sugar to rise. Thus, for example, a meal or snack having a low glycemic index produces a gradual rise in blood sugar whereas a meal or snack having a high glycemic index produces a fast rise in blood sugar. One exemplary measure of total glycemic index may be, but should not be limited to, the ratio of carbohydrates absorbed from the meal and a reference value, e.g., derived from pure sugar or white bread, over a specified time period, e.g., 2 hours. With any of the meal size or meal speed information, it will be understood that sub-process C may be configured to require a patient to enter absolute estimates as illustrated e.g., "small," or may alternatively be configured to require the patient to enter such information in relative terms, e.g., "smaller than normal."

Referring again to FIG. 5, the process 100 advances from the "NO" branch of step 106 and from completion of sub-process C to step 108 where the information collecting unit processor asks via the patient interface whether the patient wants to enter physical state information. If so, the patient responds appropriately via the input device and the process 100 advances to sub-process D. If not, the patient responds appropriately and the process 100 advances to step 110.

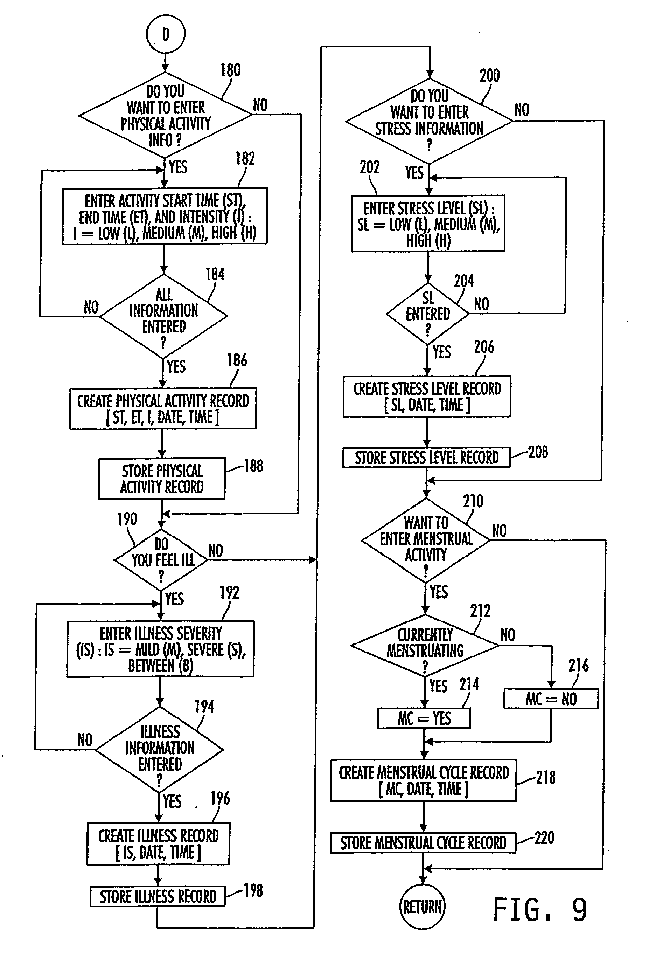

Referring now to FIG. 9, a flowchart of one illustrative embodiment of the sub-process D of FIG. 5 is shown. In the illustrated embodiment, the sub-process D begins at step 180 where the information collecting unit processor asks via the patient interface whether the patient wants to enter physical activity information. If not, the patient responds appropriately via the input device and the sub-process D advances to step 190. If, at step 180, the patient wants to enter physical activity information, the patient responds appropriately via the input device and execution of the sub-process D advances to step 182 where the information collecting unit processor instructs the patient via the patient interface to enter via the input device an activity start time, ST, and activity end time, ET, and an intensity, I, of physical activity. The patient may be presented with a menu of intensity indicators, I, and in any case the patient may enter low, L, medium, M, or high, H. Thereafter at step 184, the information collecting unit processor determines whether the patient has entered all of the information at step 182. If not, the sub-process D loops back to step 182 until the patient enters all of the physical activity information. From the "YES" branch of step 184, the sub-process D advances to step 186 where the information collecting unit processor creates a physical activity record. Illustratively, the physical activity record may take the form [ST, ET, I, DATE, TIME], where ST is the start time of the physical activity, ET is the end time of the physical activity, I is the intensity of the physical activity, DATE is the current calendar date and TIME is the current time of day. Thereafter at step 188, the information collecting unit processor stores the physical activity record in memory. It will be understood that step 182 may be modified to require the patient to include more or less information relating to the physical activity. For example, an activity duration may be substituted for the activity end time, an activity description may be required, e.g., running, walking, etc.

From step 188 and from the "NO" branch of step 180, the sub-process D advances to step 190 where the information collecting unit processor asks via the patient interface whether the patient feels ill. If not, the patient responds appropriately via the input device and the sub-process D advances to step 198. If, at step 190, the patient responds via the input device that the patient does indeed feel ill, execution of the sub-process D advances to step 192 where the information collecting unit processor instructs the patient via the patient interface to enter via the input device an illness severity, IS. The patient may enter this information directly or may be presented with a menu from which to select an illness severity value, and in any case the illness severity information may typically be entered as mild, M, severe, S, or in between mild and severe, B. Thereafter at step 194, the information collecting unit processor determines whether the patient has entered the illness severity information at step 192. If not, the sub-process D loops back to step 192 until the patient enters the illness severity information. From the "YES" branch of step 194, the sub-process D advances to step 196 where the information collecting unit processor creates an illness information record. Illustratively, the illness information record may take the form [IS, DATE, TIME], where IS corresponds to the severity of the illness (M, S or B), DATE is the current calendar date and TIME is the current time of day. Thereafter at step 198, the information collecting unit processor stores the illness information record in memory. It will be understood that step 182 may be modified to require the patient to include more or less information relating to the illness. For example, information relating to illness type and/or duration may also be entered.

From step 198 and from the "NO" branch of step 190, the sub-process D advances to step 200 where the information collecting unit processor asks via the patient interface whether the patient wants to enter patient stress information. If not, the patient responds appropriately via the input device and the sub-process D advances to step 210. If, at step 200, the patient responds via the input device that the patient wants to enter patient stress information, execution of the sub-process D advances to step 202 where the information collecting unit processor instructs the patient via the patient interface to enter via the input device a patient stress level, SL. The patient may enter this information directly or may be presented with a menu from which to select a stress level value, and in any case the patient stress information may typically be entered as low, L, medium, M, high, H. Thereafter at step 204, the information collecting unit processor determines whether the patient has entered the illness severity information at step 202. If not, the sub-process D loops back to step 202 until the patient enters the patient stress information. From the "YES" branch of step 204, the sub-process D advances to step 196 where the information collecting unit processor creates a stress level record. Illustratively, the stress level record may take the form [SL, DATE, TIME], where SL corresponds to the stress level of the patient (L, M or H), DATE is the current calendar date and TIME is the current time of day. Thereafter at step 208, the information collecting unit processor stores the stress level record in memory.

From step 208 and from the "NO" branch of step 200, the sub-process D advances to step 210 where the information collecting unit processor asks via the patient interface whether the patient wants to enter menstrual activity information. If not, the patient responds appropriately via the input device and the sub-process D returns to its calling routine. If, at step 210, the patient responds via the input device that the patient wants to enter menstrual activity information, execution of the sub-process D advances to step 212 where the information collecting unit processor asks the patient via the patient interface whether the patient is currently menstruating. If so, the patient responds appropriately via the input device and the sub-process D advances to step 214 where the information collecting unit processor sets a menstrual information variable, MC, to YES. If not, the patient responds appropriately via the input device and the sub-process D advances to step 216 where the information collecting unit processor sets the menstrual information variable, MC, to NO. From steps 214 and 216, the sub-process D advances to step 218 where the information collecting unit processor creates a menstrual cycle record. Illustratively, the menstrual cycle record may take the form [MC, DATE, TIME], where MC corresponds to the menstrual state of the patient (YES = currently menstruating, No = not currently menstruating), DATE is the current calendar date and TIME is the current time of day. Thereafter at step 220, the information collecting unit processor stores the menstrual cycle record in memory. From step 220, the sub-process D returns to its calling routine.

Referring again to FIG. 5, the process 100 advances from the "NO" branch of step 108, and from the completion of sub-process D, to step 110 where the information collecting unit processor asks the patient via the patient interface whether the patient wants to enter any comments via the input device. If so, the patient responds appropriately via the input device and execution of the process 100 advances to sub-process E. If not, the patient responds appropriately via the input device and the process 100 advances to step 112.

Referring now to FIG. 10, a flowchart is shown of one illustrative embodiment of the sub-process E of FIG. 5. In the illustrated embodiment, the sub-process E begins at step 230 where the information collecting unit processor instructs the patient via the patient interface to enter any free form comments. The patient may then respond by entering free form comments via the input device. The free form comments may include any free-form textual information that the patient wishes to enter, and such comments will typically, although not necessarily, relate to information that the patient feels may have a bearing upon the diabetes therapy that will be determined and designed by the health care professional. Alternatively or additionally, the free-form comments may include one or more words, phrases, graphs, charts or the like that are selectable from a menu. Thereafter at step 232, the information collecting unit processor determines whether the patient has finished entering comments. If not, the sub-process E loops back to step 230 until the patient indicates that the patient has finished entering comments. From the "YES" branch of step 232, the sub-process E advances to step 234 where the information collecting unit processor creates a comment record. Illustratively, the comment record may be of the form [COMMENTS, DATE, TIME], where COMMENTS is a comment field containing the free form comments entered by the patient, DATE is the current calendar date and TIME is the current time of day. Thereafter at step 236, the information collecting unit processor stores the comment record in memory. Following step 236, the sub-process E is returned to its calling routine.

Referring again to FIG. 5, the process 100 advances from the "NO" branch of step 110, and from the completion of sub-process E, to step 112 where the information collecting unit processor asks the patient via the patient interface whether the patient wants to modify any previously entered information. If so, the patient responds accordingly via the input device and the process 100 advances to sub-process F. If not, the patient responds accordingly via the input device. From the "NO" branch of step 112, and from the completion of sub-process F, the process 100 is completed.

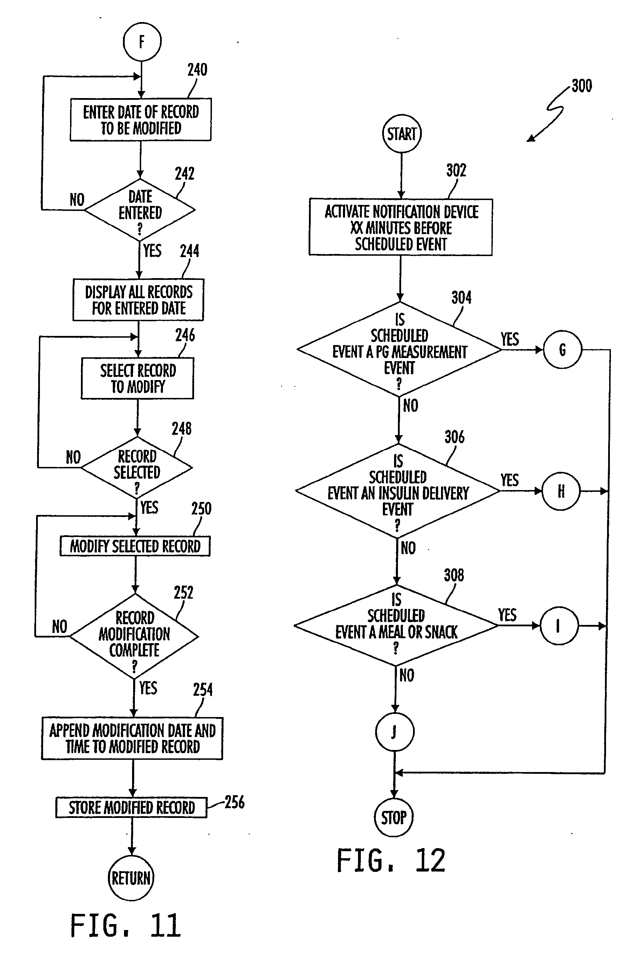

Referring now to FIG. 11, a flowchart is shown of one illustrative embodiment of the sub-process F of FIG. 5. In the illustrated embodiment, the sub-process F begins at step 240 where the information collecting unit processor instructs the patient to enter the date of the record that the patient wishes to modify. The patient then responds via the input device with an appropriate date. Alternatively, the sub-process F may be configured to allow the patient to respond at step 240 via the input device with an appropriate range of dates. Thereafter at step 242, the information collecting unit processor determines whether the patient has entered the date at step 240. If not, the sub-process F loops back to step 240 until the patient enters the date of the record to be modified. The sub-process F advances from the "YES" branch of step 242 to step 244 where the information collecting unit processor presents to the patient all records that were stored in memory for the date that the patient entered at step 240. Thereafter at step 246, the information collecting unit processor instructs the patient via the patient interface to select from the records presented at step 244 a record that the patient wishes to modify. The patient then responds via the input device by selecting a record to modify. Thereafter at step 238, the information collecting unit processor determines whether the patient has selected a record at step 246. If not, the sub-process F loops back to step 246 until the patient selects a record to modify. From the "YES" branch of step 248, the sub-process F advances to step 250 where the information collecting unit processor instructs the patient via the patient interface to modify the selected record. The patient then responds by modifying the selected record as desired. Thereafter at step 252, the information collecting unit processor determines whether the patient has modified the selected record. If not, the sub-process F loops back to step 250 until the patient indicates that the patient is done modifying the selected record. From the "YES" branch of step 252, the sub-process F advances to step 254 where the information collecting unity processor appends a modification date and time to the modified record. The modification date and time correspond to the current calendar date and current time of day. Thereafter at step 256, the information collecting unit processor stores the modified record in memory, and the sub-process F then returns to its calling routine. It will be understood that the sub-process F may be alternatively or additionally designed to allow the patient to search records for modification according to other search criteria. Examples of such other search criteria include, but are not limited to, meal type, meal composition, exercise information, illness information, information relating to therapy related medication being taken by the patient, and the like. Modifications to the sub-process F to allow searching of records according to one or more such alternate criterion would be a mechanical step for a skilled programmer.

Referring now to FIG. 12, a flowchart is shown of an alternate or additional illustrative process 300 for collecting patient information from which diabetes therapy may be determined using either of the systems of FIGS. 1 and 4. In the illustrated embodiment, the process 300 begins at step 302 where the information collecting unit processor activates the patient notification device XX minutes before a scheduled event, where "XX" may be any positive integer. Alternatively, "XX" may be or include any number of seconds. Alternatively, "XX" may correspond to a specified time of day. In any case, activation of the notification device in this manner alerts the patient that the patient will shortly be required to perform one or more acts, and/or to enter patient information into the information collecting unit. Thereafter at step 304, the information collecting unit processor determines whether the scheduled event is a patient glucose measurement event. If so, the process 300 advances to a sub-process G. If not, the process 300 advances to step 306.

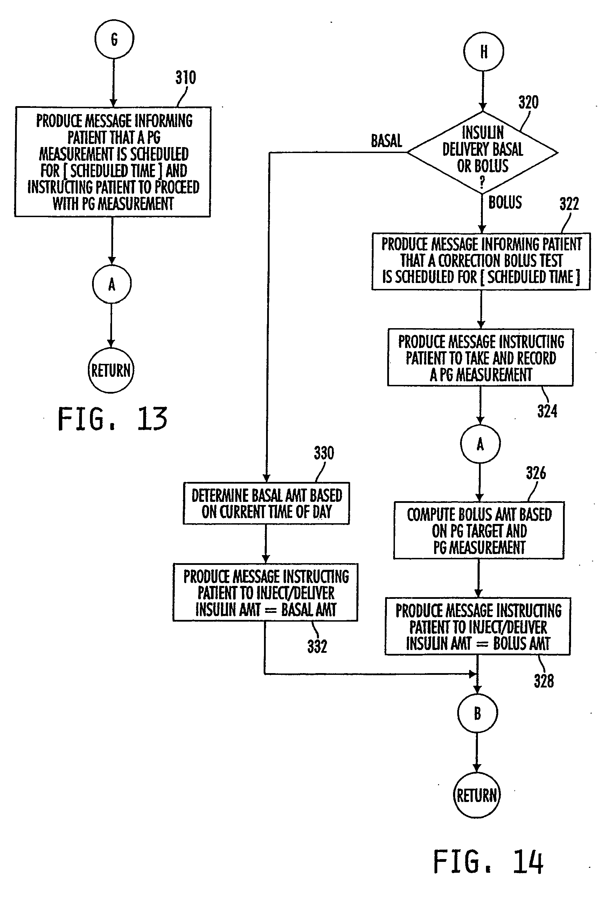

Referring now to FIG. 13, a flowchart is shown of one illustrative embodiment of the sub-process G. In the illustrated embodiment, the sub-process G begins at step 310 where the information collecting unit processor produces a message informing the patient that a patient glucose measurement is scheduled for [SCHEDULED TIME] and instructing the patient to proceed with taking a patient glucose measurement, where [SCHEDULED TIME] corresponds to the programmed time that the scheduled patient glucose measurement event is to take place. Following step 310, the sub-process G advances to the sub-process A of FIG. 6 and then returns to its calling routine following completion of the sub-process A. It will be understood that in embodiments of the system 10 of FIG. 1 where the patient glucose measurement device 34 is configured to automatically transfer patient glucose information to the information collecting unit, step 304 and sub-process G are not necessary and may be omitted.

Referring again to FIG. 12, the process 300 advances from the "NO" branch of step 304 and from completion of the sub-process G to step 306 where the information collecting unit processor determines whether the scheduled event is an insulin delivery event. If so, the processor 300 advances to a sub-process H, and if not the process 300 advances to step 308.

Referring now to FIG. 14, a flowchart is shown of one illustrative embodiment of the sub-process H of FIG. 12. In the illustrated embodiment, the sub-process H begins at step 320 where the information collecting unit processor asks the patient via the patient interface whether the insulin delivery event corresponds to a basal insulin delivery or a bolus insulin delivery. If the patient responds that the insulin delivery event corresponds to a bolus insulin delivery, the sub-process H advances to step 322 where the information collecting unit processor produces a message via the patient interface informing the patient that a correction bolus test is scheduled for [SCHEDULED TIME], where [SCHEDULED TIME] corresponds to the time at which the patient is to determine whether a correction bolus is necessary and, if so, in what amount. From step 322, the sub-process H advances to step 324 where the information collecting unit processor produces a message via the patient interface instructing the patient to take and record a patient glucose measurement. Thereafter, the sub-process H advances to the sub-process A of FIG. 6 where the information collecting unit processor guides the patient through creation and storage of a patient glucose measurement record as described hereinabove. Thereafter when the sub-process A is completed, the sub-process H advances to step 326 where the information collecting unit processor is configured to compute an insulin bolus amount based, at least in part, on a predetermined glucose target and on the patient glucose measurement just taken pursuant to step 324. Thereafter at step 328, the information collecting unit processor produces a message via the patient interface instructing the patient to inject, or otherwise deliver, insulin in the amount of the bolus amount computed at step 326. In an alternate embodiment, step 326 may be omitted and the patient may instead compute the insulin bolus amount based, at least in part, on the patient glucose target and on the recently-taken patient glucose measurement. In any case, following step 328, the sub-process H advances to the sub-process B of FIG. 7 where the information collecting unit processor guides the patient through creation and storage of an insulin delivery record as described hereinabove.

If, at step 320, the patient responds that the insulin delivery event corresponds to a basal insulin delivery event, the sub-process H advances to step 330 where the information collecting unit processor is configured to determine a basal amount of insulin based, at least in part, on the current time of day. Thereafter at step 332, the information collecting unit processor produces a message via the patient interface instructing the patient to inject or otherwise deliver insulin in the amount of the insulin basal amount determined at step 330. In an alternative embodiment, step 330 may be omitted, and the patient may instead determine the basal insulin amount based, at least in part, on the current time of day. In any case, the sub-process H advances from step 332 to the sub-process B of FIG. 7 where the information collecting unit processor guides the patient through creation and storage of an insulin delivery record as described hereinabove. Thereafter, the sub-process H is returned to its calling routine.

Referring again to FIG. 12, the process 300 advances from the "NO" branch of step 306 to step 308 where the information collecting unit processor determines whether the scheduled event corresponds to a meal or snack. If so, the process 300 advances to a sub-process, I, and otherwise advances to a sub-process J. The process 300 stops after execution of any of the sub-processes G, H, I and J.

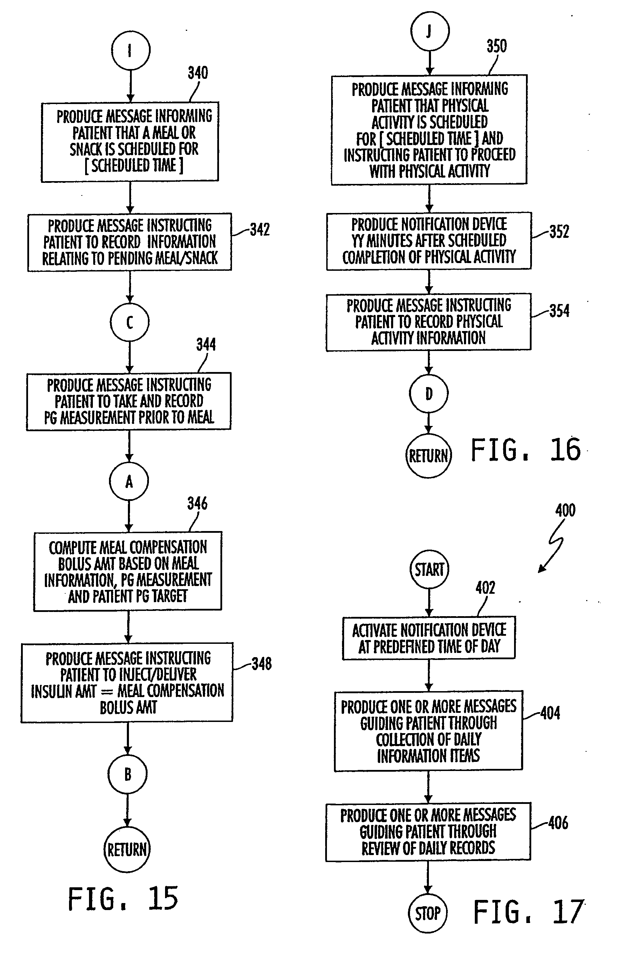

Referring now to FIG. 15, a flowchart is shown of one illustrative embodiment of the sub-process I of FIG. 12. In the illustrated embodiment, the sub-process I begins at step 340 where the information collecting unit processor produces a message via the patient interface informing the patient that a meal or snack is scheduled for [SCHEDULED TIME], where [SCHEDULED TIME] corresponds to the time at which the patient is scheduled to consume the meal or snack. Thereafter at step 342, the information collecting unit processor produces a message via the patient interface instructing the patient to record information relating to the pending meal or snack. Thereafter, the sub-process I advances to the sub-process C of FIG. 8 where the information collecting unit processor guides the patient through the creation and storage of meal or snack-related information. Following completion of the sub-process C, the information collecting unit processor is operable at step 334 to produce a message via the patient interface instructing the patient to take and record a patient glucose measurement prior to consuming the scheduled meal or snack. Following step 344, the sub-process I advances to the sub-process A where the information collecting unit processor guides the patient through the creation and recording of the patient glucose measurement information. Following completion of the sub-process A, the information collecting unit processor is operable at step 346 to compute a meal compensation bolus amount based, at least in part, on the meal information just entered by the patient, the patient glucose measurement and on a predefined glucose target. Thereafter at step 348, the information collecting unit processor produces a message via the patient interface instructing the patient to inject, or otherwise deliver, insulin in the amount of the meal compensation bolus amount computed at step 346. Alternatively, step 346 may be omitted, and the patient may instead compute the meal compensation bolus amount. In any case, step 348 advances to the sub-process B of FIG. 7 where the information collecting unit processor guides the patient through the creation and storage of the insulin delivery information. Thereafter, the sub-process I is returned to its calling routine.

Referring now to FIG. 16, a flowchart is shown of one illustrative embodiment of the sub-process J of FIG. 12. In accordance with the process 300 of FIG. 12, if the scheduled event does not correspond to a patient glucose measurement event, an insulin delivery event or a meal or a snack, the scheduled event must then correspond to a scheduled physical activity. In the illustrated embodiment of the sub-process J, the information collecting unit processor therefore produces a message at step 350 via the patient interface informing the patient that a physical activity is scheduled for [SCHEDULED TIME] and instructing the patient to proceed with the scheduled physical activity, where [SCHEDULED TIME] corresponds to the time of day at which the patient is scheduled to undertake the physical activity. From step 350, the sub-process J advances to step 352 where the information collecting unit processor activates the patient notification device YY minutes after the scheduled completion of the physical activity, where "YY" may be any positive integer. Alternatively, "YY" may be or include any number of seconds. Alternatively still, "YY" may correspond to a predefined time of day. In any case, the information collecting unit processor is thereafter configured at step 354 to produce a message via the patient interface instructing the patient to record details of the physical activity information. The sub-process J advances from step 354 to the sub-process D where the information collecting unit processor guides the patient through the creation and storing of the physical activity information. It will be understood that for purposes of the sub-process J, the sub-process D may be modified to execute only steps 182-188 of the sub-process D before returning to the sub-process J. In any case, the sub-process J returns after completion of the sub-process D to its calling routine. It will be understood that the process 300 of FIG. 12 may be modified to include notification of other therapy-relevant events. Examples of such other therapy-relevant events include, but are not limited to, scheduled administering of medication, scheduled visits to a physician, and the like.

Referring now to FIG. 17, a flowchart is shown of another alternate or additional illustrative process 400 for collecting patient information for which diabetes therapy may be determined using either of the systems of FIGS. 1 and 4. In the illustrated embodiment, the process 400 begins at step 402 where the information collecting unit processor activates the patient notification device each day at a predetermined time. Activation of the notification device in this manner alerts the patient that the patient is to enter information into the system 10 or 10'. Thereafter at step 404, the information collecting unit processor produces one or more messages via the patient interface guiding the patient through the collection and storage of patient information that may be entered on a daily basis, e.g., once per day. In accordance with step 404, one or more of the patient information records is predefined and pre-existing in memory, and contains default patient information values. The information collecting unit processor then guides the patient through the default records and instructs the patient via the patient interface to modify the content of the default records as appropriate.

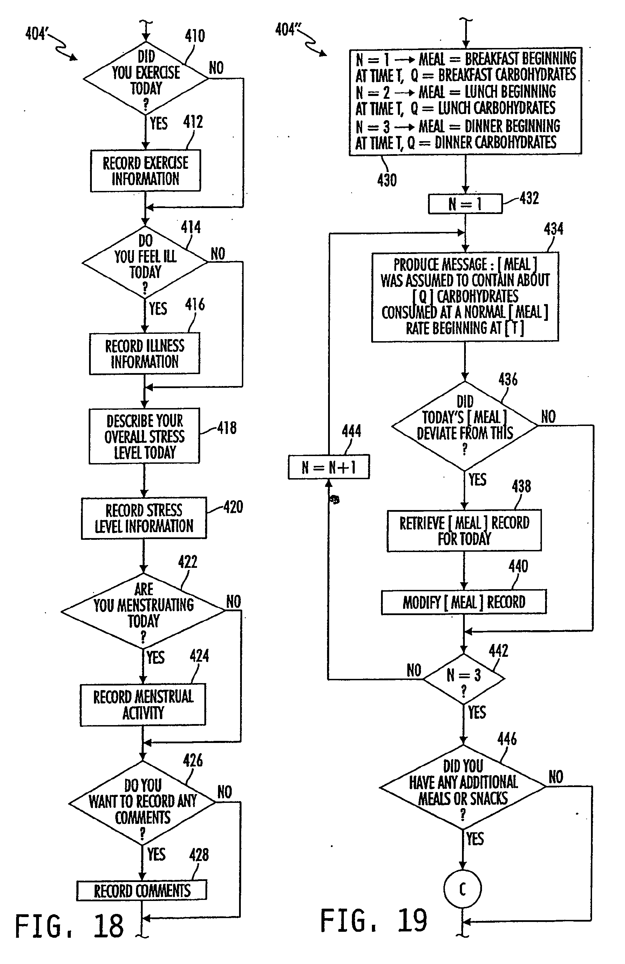

Referring now to FIG. 18, a flowchart is shown of one illustrative process 404' for accomplishing step 404 of FIG. 17. In the illustrated embodiment, the process 404' begins at step 410 where the information collecting unit processor asks the patient via the patient interface whether the patient exercised during the present day. If the patient responds via the input device that the patient did exercise during the present day, the process 404' advances to step 412 where the information collecting unit processor guides the patient through the collecting and recording of patient exercise that occurred throughout the day. Illustratively, step 412 may be accomplished by guiding the patient through steps identical to or similar to steps 182-188 of the sub-process D of FIG. 9 a number of times until the patient has entered all exercise information for the day. From step 412 and from the "NO" branch of step 410, process 404' advances to step 414 where the information collecting unit processor asks the patient via the patient interface whether the patient feels ill during the present day. If the patient responds via the input device that the patient does feel ill, the process 404' advances to step 416 where the information collecting unit processor guides the patient through the collecting and recording of patient illness information. Illustratively, step 416 may be accomplished by guiding the patient through steps identical to or similar to steps 192-198 of the sub-process D of FIG. 9.

From step 416 and from the "NO" branch of step 414, the process 404' advances to step 418 where the information collecting unit processor produces a message via the patient interface instructing the patient to describe the patient's overall stress during the present day. The patient responds appropriate via the input device and the process 404' thereafter advances to step 420 where the information collecting unit processor records the stress level information entered by the patient. Illustratively, steps 418-420 may be accomplished by guiding the patient through steps identical to or similar to steps 202-208 of the sub-process D of FIG. 9.

From step 420, the process 404' advances to step 422 where the information collecting unit processor asks the patient via the patient interface whether the patient is menstruating during the present day. If the patient responds via the input device that the patient is menstruating, the process 404' advances to step 424 where the information collecting unit processor guides the patient through the collecting and recording of patient menstrual cycle information. Illustratively, step 424 may be accomplished by guiding the patient through steps identical to or similar to steps 212-220 of the sub-process D of FIG. 9.

From step 424 and from the "NO" branch of step 422, the process 404' advances to step 426 where the information collecting unit processor asks the patient via the patient interface whether the patient wants to enter and record any comments. If so, the patient responds accordingly and the process 404' advances to step 428 where the information collecting unit processor guides the patient through the collecting and recording of patient comments. Illustratively, step 428 may be accomplished by guiding the patient through a process that is identical to or similar to sub-process E of FIG. 10. Thereafter, and after the "NO" branch of step 426, the process 404' is completed.

Referring now to FIG. 19, a flowchart is shown of another illustrative process 404" for executing the step 404 of the process 400 of FIG. 17. With the process 404", the information collecting unit processor presumes that each meal consumed by the patient during the present day was normal-sized, contained an amount of carbohydrates that is typical for the meal, was consumed at a rate that is normal for the meal, and that began at or near a specified time. The process 404" then guides the patient through a process for modifying these presumptions based on actual meal information for each of the various meals consumed by the patient during the present day. In the illustrated embodiment, the process 404" begins at step 430 where meal type, meal starting time and carbohydrate content parameters are defined for the various meals consumed by the patient during the day. For example, if a counter "N" is equal to 1, the meal corresponds to breakfast beginning at time T, and Q corresponds to the typical amount of carbohydrates contained in a typical breakfast for the patient. If the counter "N" equals 2, the meal corresponds to lunch beginning at time T, and Q corresponds to a typical carbohydrate content for a typical lunch for the patient. If the counter "N" equals 3, the meal corresponds to dinner beginning at time T, and Q corresponds to a typical carbohydrate content of a typical dinner consumed by the patient. Following step 430, the process 404" advances to step 432 where the counter "N" is set equal to 1. Thereafter at step 434, the information collecting unit processor produces a message via the patient interface informing the patient that the [MEAL], e.g., breakfast, lunch or dinner, was assumed to contain about [Q] carbohydrates, and that the meal was assumed to be consumed at a normal [MEAL] rate beginning at time [T]. Thus, for each meal, the information collecting unit processor presents to the patient via the patient interface a pre-defined meal record containing default meal values. It will be understood that the default meal values may include more or less meal information, and examples of more meal information include, but are not limited to, carbohydrate amount, meal composition, protein amount, fat amount, glycemic index information, and the like.

Following step 434, the process 404" advances to step 436 where the information collecting unit processor asks the patient via the patient interface whether today's [MEAL], e.g., breakfast, lunch or dinner, deviated from the displayed default values. If the patient indicates via the input device that today's [MEAL] did deviate from the default meal values, the process 404" advances to step 438 where the information collecting unit processor retrieves the [MEAL] record for that day. Thereafter at step 440, the information collecting unit processor instructs the patient via the patient interface to modify the [MEAL] record in accordance with meal information that corresponds to the actual [MEAL] that was consumed by the patient. The patient then responds by appropriately modifying the [MEAL] record via the input device. Following step 440, and from the "NO" branch of step 436, the process 404" advances to step 442 where the information collecting unit processor determines whether all three meals have been processed. If not, the counter "N" is incremented at step 444, and the process 404" loops back to step 434. If, at step 442, the information collecting unit processor determines that all meal records for the current day have been processed, the process 404" advances to step 446 where the information collecting unit processor asks the patient via the patient interface whether the patient had any additional meals or snacks. If the patient indicates via the input device that the patient did have meals or snacks in addition to those already presented to the patient via the process 404", the process 404" advances to the sub-process C of FIG. 8 where the information collecting unit processor guides the patient through the creation and storing of information relating to any such additional meals or snacks. Upon completion of the sub-process C, and from the "NO" branch of step 446, the process 404" is concluded.

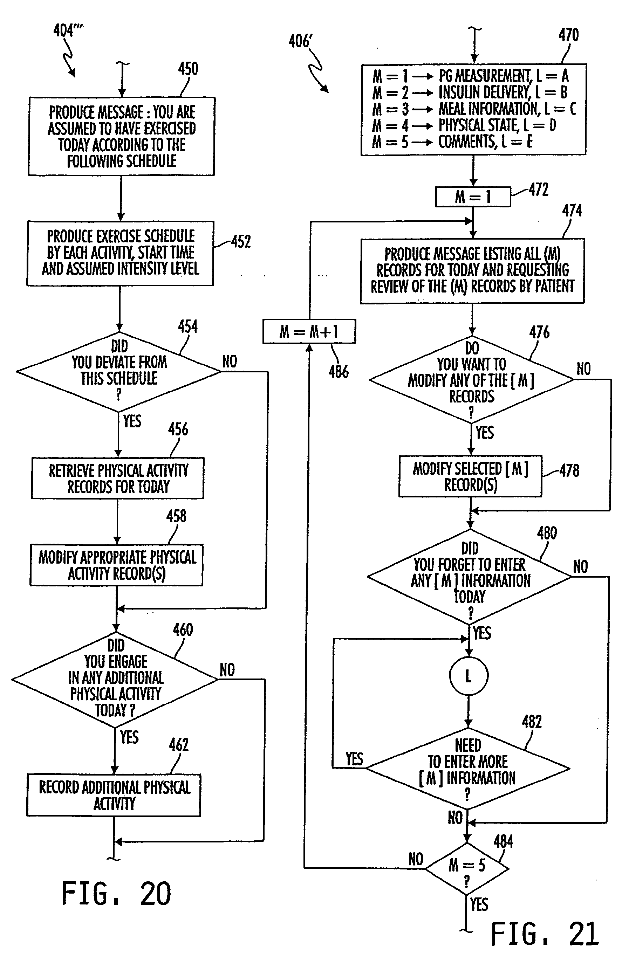

Referring now to FIG. 20, a flowchart is shown of yet another illustrative process 404''' for executing the step 404 of the process 400 of FIG. 17. The process 404''' may be used alone or in addition to the process 404" illustrated in FIG. 19. With the process 404"', the information collecting unit processor presumes that the patient exercised during the present day according to a predefined exercise schedule. The process 404''' then guides the patient through a process for modifying these presumptions based on actual exercise undertaken by the patient during the present day. In the illustrated embodiment, the process 404"' begins at steps 450 where the information collecting unit processor produces a message via the patient interface informing the patient that the patient is assumed to have exercised during the present day according to the following exercise schedule. Thereafter at step 452, the information collecting unit processor is configured to produce via the patient interface a predefined exercise schedule that is defined for the patient for the present day and that is stored in memory. The predefined exercise schedule may include, for example, but should not be limited to each activity, corresponding start time and assumed exercise intensity level, e.g., low, medium or high. Thus, for each scheduled patient activity, the information collecting unit processor presents to the patient via the patient interface a pre-defined exercise record containing default start times and default intensity level values. It will be understood that the pre-defined exercise records may contain more or less exercise information as described hereinabove with respect to the sub-process D of FIG. 9.

Following step 452, the process 404''' advances to step 454 where the information collecting unit processor asks the patient via the patient interface whether the patient deviated from the default exercise schedule presented via the patient interface at step 452. If the patient indicates via the input device that the patient did deviate from the default exercise schedule, the process 404''' advances to step 456 where the information collecting unit processor retrieves all of the physical activity records for the present day. Thereafter at step 458, the information collecting unit processor instructs the patient via the patient interface to modify the appropriate physical activity record(s) in accordance with actual physical activity undertaken by the patient during the present day. The patient then responds by appropriately modifying the physical activity record(s) via the input device. Following step 458, and from the "NO" branch of step 454, the process 404''' advances to step 460 where the information collecting unit processor asks the patient via the patient interface whether the patient engaged in any additional physical activities during the present day that were not listed in the default exercise schedule presented to the patient at step 452. If the patient indicates via the input device that the patient did engage in one or more physical activities in addition to those already presented to the patient at step 452, the process 404''' advances to step 462 where the information collecting unit processor guides the patient through creation and storing of one or more corresponding additional physical activity information records. Illustratively, step 462 may be accomplished via execution of steps similar or identical to steps 180-188 of the sub-process D of FIG. 9. Upon completion of step 462, and from the "NO" branch of step 460, the process 404''' is concluded.

The process 400 of FIG. 17 may further include another step in which the patient periodically reviews, e.g., on a daily basis, the patient information records stored in memory for that period and modifies, as appropriate, the stored information as a corrective or information updating measure. In the illustrated embodiment, for example, the process 400 advances from step 404 to step 406 where the information collecting unit processor produces one or more messages via the patient interface that guide the patient through review, and possible modification, of the daily records. After completion of step 406, the process 400 is completed. Alternatively, step 406 may be provided as a stand alone process that may be executed by the information collecting unit processor independently of, or in addition to, any of the processes illustrated and described herein. In any case, referring now to FIG. 19, a flowchart is shown of an illustrative process 406' for executing step 406. With the process 406', the patient information records for each day are grouped for review according to patient information record type. For example, as illustrated in the first step 470 of the process 406', if a counter "M" is equal to 1, the patient information record type corresponds to patient glucose measurement records and a sub-process identifier, L, is set equal to "A" which is the sub-process of FIG. 6 that is configured to guide the patient through the creation and storing in memory of patient glucose measurement information. If the counter "M" is equal to 2, the patient information record type corresponds to patient insulin delivery records and a sub-process identifier, L, is set equal to "B" which is the sub-process of FIG. 7 that is configured to guide the patient through the creation and storing in memory of patient insulin delivery information. If the counter "M" is equal to 3, the patient information record type corresponds to patient meal information records and a sub-process identifier, L, is set equal to "C" which is the sub-process of FIG. 8 that is configured to guide the patient through the creation and storing in memory of patient meal information. If the counter "M" is equal to 4, the patient information record type corresponds to patient physical state records and a sub-process identifier, L, is set equal to "D" which is the sub-process of FIG. 9 that is configured to guide the patient through the creation and storing in memory of patient physical state information including, for example, physical activities, illness, stress, menstrual cycle and the like. Finally, if the counter "M" is equal to 5, the patient information record type corresponds to patient comment records and a sub-process identifier, L, is set equal to "E" which is the sub-process of FIG. 10 that is configured to guide the patient through the creation and storing in memory of patient comments. Following step 470, the process 406' advances to step 472 where the counter "M" is set equal to 1. Thereafter at step 474, the information collecting unit processor produces a message via the patient interface listing all "M" records, e.g., all patient glucose measurement records, for the review period, e.g., for the current day, and requesting review of all of the "M" records by the patient. Thus, for each group of patient information records, the information collecting unit processor presents to the patient via the patient interface a listing of the corresponding records for that group.

Following step 474, the process 406' advances to step 476 where the information collecting unit processor asks the patient via the patient interface whether the patient wants to modify any of the [M] records. If the patient wishes to modify any one or more of the [M] records, the patient selects via the input device appropriate one(s) of the displayed [M] records and then proceeds to modify the selected [M] record(s) at step 478. From step 478 and from the "NO" branch of step 476, the process 406' advances to step 480 where the information collecting processor asks the patient via the patient interface whether the patient may have forgotten or otherwise neglected to enter any [M] information for the current day. If the patient responds via the input device that the patient did forget or neglect to enter [M] information for the current day, the process 406' advances to the sub-process L, where "L" is defined by steps 470, 472 and 486 as will be described hereinafter. After completion of the sub-process L, the process 406' advances to step 482 where the information collecting unit processor asks the patient via the patient interface whether the patient needs or wants to enter more [M] information. If so, the patient responds accordingly via the input device and the process 406' loops back to the "YES" branch of step 480. If, at step 482, the patient does not wish to enter additional [M] information, the patient responds accordingly via the input device and the process 406' advances to step 484 where the information collecting unit processor determines the value of "M." If "M" is not equal to five, the process 406' advances to step 486 where "M" is incremented by one before looping back to step 474. If, at step 484, the information collecting unit processor determines that M=5, the process 406' is concluded.

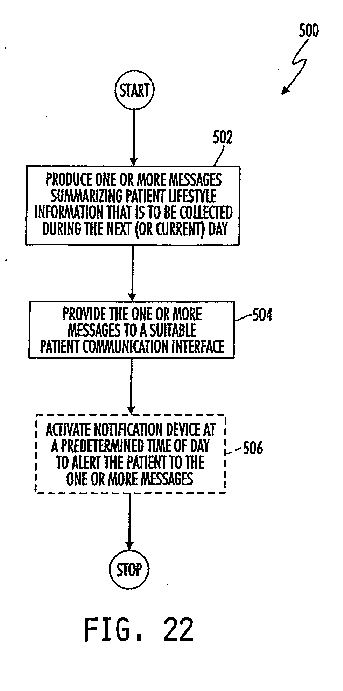

Referring now to FIG. 22, a flowchart of one illustrative process 500 is shown for providing a summary of patient lifestyle information to be collected during the next or current day. The process 500 may be executed by the processor of any of the one or more patient-side electronic devices 12 or the HCP electronic device, and any information transfer technique described herein may be used to provide the summary information to the patient via any one or more of the patient communication interfaces described herein. The process 500 begins at step 502 where the processor executing the process 500 is operable to produce one or more messages summarizing the patient lifestyle information that is expected to be collected by the patient during the next day, in cases where the patient reviews the summary information in the evening prior to bed time, or that is expected to be collected by the patient during the current day, in cases where the patient reviews the summary information in the morning of the current day. From step 502, the process 500 advances to step 504 where the one or more messages is provided to the patient via a suitable one or more of the patient communication interfaces described hereinabove. Examples of suitable patient communication interfaces include, but are not limited to, a display unit, a patient-accessible web site, a pre-recorded voice message, voice mail, etc.

In one embodiment, the process 500 may be configured to require the patient to access the patient communication interface for the summary information. In this embodiment, the patient may, for example, access a web site to view the summary information, manipulate one of the patient-side electronic devices 12 to display the summary information, access a dial-up service or voice mail for a pre-recorded voice message of the summary information, or the like. Alternatively, the process 500 may be configured to notify the patient when such summary information is available or to remind the patient to review the provided summary information. In this embodiment, the process 500 includes the optional step 506 (shown in dashed-line representation) where the processor executing the process 500 activates an appropriate one or more of the notification devices at a predetermined time of day to alert the patient to the summary information. The alerted patient may then access the summary information via an appropriate patient communication interface. In any case, the process 500 terminates after step 504 in the former embodiment, and after step 506 in the later embodiment.

Many of the various processes and sub-processes illustrated and described hereinabove include steps where the information collecting unit processor asks the patient for information or instructs the patient to enter specified information, followed by steps where the information collecting unit processor determines whether the patient has entered the requested or specified information. Although not specifically shown in the flowcharts, those skilled in the art will recognize that these processes and sub-processes will typically include conventional techniques for requiring the patient to inform the information collecting unit processor when the patient is finished entering information. An example of one such conventional technique for display-type patient interfaces includes, but should not be limited to, producing a graphical "ACCEPT,", "COMPLETE," "FINISHED," or "OK" icon on the patient interface which, when selected by the patient, informs the information collecting unit processor that the patient is finished entering the requested or specified information. Those skilled in the art will recognize other such conventional techniques for display-type and other patient interfaces, and any such other conventional techniques are contemplated by the present disclosure. The steps where the information collecting unit processor determines whether the patient has entered the requested or specified information may illustratively further include a timeout mechanism configured to direct the corresponding process or sub-process to a specified step or state if the patient does not completely enter the requested or specified information within a specified time period. The processes and sub-processes illustrated and described herein may further include one or more steps that allow the patient to modify previously entered information, or to append new and/or perhaps more accurate information onto the previously entered information. This optional feature provides the patient with the ability to modify previously entered information such as in cases where, for example, meal-related information was entered prior to or during ingestion of the meal, to subsequently reflect any deviations in actual meal ingestion from that which was expected or estimated at the time the information was entered. For example, a scheduled meal may be skipped or delayed, more or less of the meal may have actually been consumed as compared with what was previously estimated, and/or the composition of the meal may have varied from what was previously estimated. In any case, modifying the processes and sub-processes illustrated and described herein to include any of the features just described would be a mechanical step for a skilled programmer.

It will be understood that while the patient information collection examples have been described herein in the context of daily collection of information, such information need not be strictly collected on a daily basis. Generally, the frequency and periodicity of patient information collection will be determined by the health care professional, and may vary between patients. For example, the health care professional may require one patient to collect lifestyle information on a frequent basis each day, and for another patient the health care professional may require collection of lifestyle information only on weekends, or only on a certain day or days and only within a certain time window or windows.

It will also be understood that the system 10 for collecting patient information may be used to establish a diabetes therapy and/or to modify an existing diabetes therapy. It is envisioned, for example, that the one or more patient-side electronic devices 12 may include at least one device for collecting patient information and at least one for managing a HCP-designed therapy. These may be incorporated, for example, into a single device. After a diabetes therapy has been established, the health care professional may determine that it is appropriate for the patient to collect additional lifestyle information for a specified period of time, and may accordingly configure the system 10 so that the patient will collect such additional lifestyle information while also undergoing the established diabetes therapy. The health care professional may then determined, based on at least some of the additional lifestyle information that has been collected, to modify the diabetes therapy. This cycle may be repeated any number of times, and/or be carried out periodically, e.g., yearly, or at any time deemed appropriate by the health care professional.

While the invention has been illustrated and described in detail in the foregoing drawings and description, the same is to be considered as illustrative and not restrictive in character, it being understood that only illustrative embodiments thereof have been shown and described and that all changes and modifications that come within the spirit of the invention are desired to be protected.

## Claims

1. A system for collecting patient information from which diabetes therapy may be determined, the system comprising:
a patient interface device,
a patient notification device,
an input device for entering patient information, and
an information collecting unit including a processor electrically coupled to a memory having stored therein at least one algorithm executable by the processor to activate the patient notification device followed by presenting instructions to the patient via the patient interface device to collect specified information from the patient via the input device.

2. A system of claim 1, **characterized in that** the system comprising a glucose measuring device configured to conduct at least two measurements of glucose concentration of a body fluid of a patient,
whereas at a predetermined time to alert the patient to conduct at least one of the at least two measurement of glucose concentration of the bodily fluid of the patient using the glucose measuring device and a patient information records stored in the memory including the at least two measurements of glucose concentration and at least one of information relating to carbohydrate, amount of food consumed by the patient, composition of food, therapy related medication taken by the patient or the physical state of the patient whereby a therapy can be designed or modified for a patient based on an analysis of this data.

3. A system of claim 2, **characterized in that** an information collecting unit with a processor determining whether a patient has entered the requested information of the patient information record and a time out mechanism configured to direct the process or sub-process for data entering of a patient information record to a specified step or state if the patient does not completely enter the requested or specified information within a specific time period.

4. A system of claim 1, whereby the notification device is or includes a pager.

5. A system for collecting patient information from which diabetes therapy may be determined, the system comprising:
a patient interface device,
an input device for modifying patient information, and
an information collecting unit including a processor electrically coupled to a memory having stored therein at least one algorithm executable by the processor to present default values of at least some of the patient information to the patient via the patient interface device, followed by presenting instructions to the patient via the patient interface device to replace any of the default values with corresponding actual values of patient information via the input device.

6. The system of claim 5 wherein the patient information is stored in the memory of the information collecting unit,
and wherein the patient information includes information relating to timing and carbohydrate amount of food consumed by the patient, the patient's glucose concentration, insulin received by the patient and physical state of the patient.

7. A system for collecting patient information from which diabetes therapy may be determined, the system comprising:
a patient interface device,
a patient notification device,
an input device for entering patient information, and
an information collecting unit including a processor electrically coupled to a memory having stored therein at least one algorithm executable by the processor to activate the patient notification device followed by presenting a list of patient information records for the current day to the patient via the patient interface device, followed by presenting instructions to the patient via the patient interface device to correct information in any of the listed patient information records via the input device.

8. The system of claim 7 wherein the patient information records are stored in the memory of the information collecting unit,
and wherein the patient information records include information relating to timing and carbohydrate amount of food consumed by the patient, the patient's glucose concentration, insulin received by the patient and physical state of the patient.

9. A method for collecting patient information from which diabetes therapy may be determined, the method comprising:
programming an information collecting device with instructions specific to a patient for entering specified patient information therein,
activating a patient notification device at one or more predetermined times to alert the patient to enter the specified patient information into the information collecting device according to the instructions, and
using at least some of the patient information entered into the information collecting device to determine a diabetes therapy for the patient.
